# FASCICULE DE BREVET EUROPEEN

(11) **EP 1 771 425 B1**
(45) Date de publication et mention de la délivrance du brevet: **14.05.2008**
(21) Numéro de dépôt: 05786078.5
(22) Date de dépôt: 05.07.2005
(51) Int. Cl.: C07D 277/40, C07D 277/44, C07D 417/12, A61K 31/551, A61K 31/427, A61K 31/496, A61P 37/00, A61P 29/00, A61P 31/00, A61P 9/00, A61P 3/04

(54) **DERIVES DE 2-CARBAMIDE-4-PHENYLTHIAZOLE, LEUR PREPARATION ET LEUR APPLICATION EN THERAPEUTIQUE**
2-CARBAMID-4-PHENYLTHIAZOL-DERIVATE, DEREN HERSTELLUNG UND THERAPEUTISCHE VERWENDUNG
2-CARBAMIDE-4-PHENYLTHIAZOLE DERIVATIVES, PREPARATION THEREOF AND THERAPEUTIC USE THEREOF

(30) Priorité: 09.07.2004 FR 0407695
(43) Date de publication de la demande: 11.04.2007
(73) Titulaire: Sanofi-Aventis, 75013 Paris (FR)
(72) Inventeur: CASELLAS, Pierre, F-34090 Montpellier (FR); FLOUTARD, Daniel, F-34980 Combaillaux (FR); FRAISSE, Pierre c/o sanofi-aventis, Département Brevets, 75013 Paris (FR); JEGHAM, Samir, F-34980 Montferrier-sur-Lez (FR)
(74) Mandataire: Le Pennec, Magali
(86) Numéro de dépôt international: PCT/FR2005/001729
(87) Numéro de publication internationale: WO 2006/016039

(56) Documents cités:
- EP-A- 0 518 731
- EP-A- 0 519 449
- US-B1- 6 506 751
- SHELLEY ALLEN ET AL.: "Discovery and SAR of trisubstituted thiazolidinones as CCR4 antagonists" BIOORGANIC AND MEDICINAL CHEMISTRY LETTERS, vol. 14, avril 2004 (2004-04), pages 1619-1624, XP002318771

## Description

L'invention se rapporte à des dérivés de 2-carbamide-4-phénylthiazole, à leur préparation et à leur application en thérapeutique.

L'invention a pour objet des composés répondant à la formule (I) ci-après : 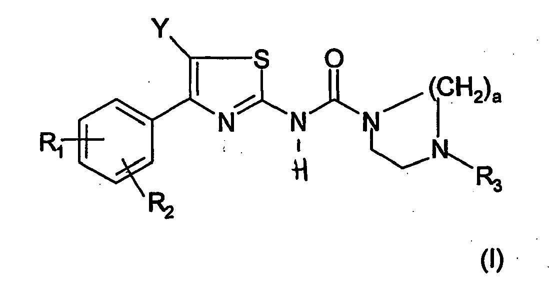 dans laquelle :
- (i) R₁ est sélectionné dans le groupe constitué par H, halogène, (C₁-C₈)alkyle, trifluoro(C₁-C₄)alkyle, -OH, -O-(C₁-C₈)alkyle, -O-trifluoro(C₁-C₈)alkyle,-O-(C₃-C₁₀)cycloalkyl(C₁-C₈)alkyle, -O-(C₃-C₁₀)cycloalkyle, -O-CH₂-CH=CH₂, et (C₁-C₄)alkylthio ;
- (ii) R₂ est sélectionné dans le groupe constitué par H, halogène, -OH, (C₁-C₈)alkyle, trifluoro(C₁-C₄)alkyle, perfluoro(C₁-C₄)alkyle, (C₃-C₁₀)cycloalkyle,-O-(C₁-C₈)alkyle, -O-(C₃-C₁₀)cycloalkyl(C₁-C₈)alkyle, -O-(C₃-C₁₀)cycloalkyle, -O-CH₂-CH=CH₂ , et (C₃-C₈)cycloalkyl(C₁-C₈)alkyle ;
- (iii) Y représente un atome d'hydrogène ou un halogène ;
- (iv) R₃ représente :
   **a1)** un groupe de formule -(CH₂)ₚ-A
      dans laquelle p représente 0, 1, 2, 3 ou 4 et :
      - lorsque p représente 2, 3 ou 4, A représente un groupe de formule : 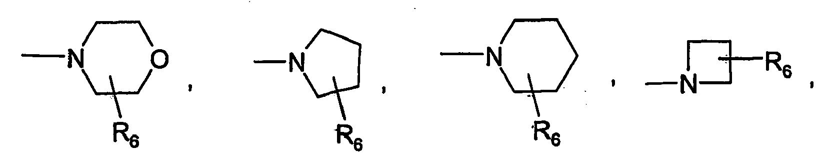 ou -NR₄R₅,
         dans laquelle R₆ est sélectionné dans le groupe constitué par H, F, (C₁-C₄)alkyle, -(CH₂)ₙOH, -(CH₂)ₙO(C₁-C₄)alkyle, et -(CH₂)ₙNR₄R₅, où n représente 0, 1 ou 2 et R₄ et R₅ représentent indépendamment l'un de l'autre un atome d'hydrogène, un groupe (C₁-C₈)alkyle, -CO(C₁-C₄)alkyle, ou -CO-O-(C₁-C₉)alkyle;
      - ou, lorsque p représente 1, 2, 3 ou 4, A représente un groupe de formule : 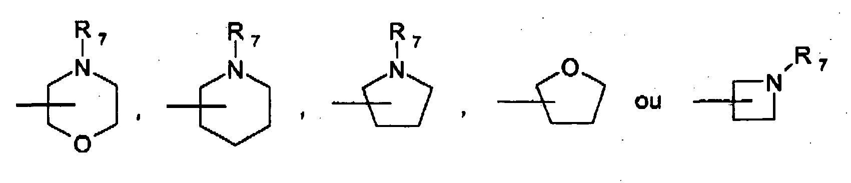 dans laquelle R₇ est sélectionné dans le groupe constitué par H, (C₁-C₈)alkyle,-CO-(C₁-C₈)alkyle, benzyle, -CO-O-(C₁-C₈)alkyle, -CO-O-benzyle, -CO-phenyl,-CO-hétéroaryle, -CO-(C₃-C₁₀)cycloalkyle, -SO₂-(C₁-C₈)alkyle, -SO₂-(C₃-C₈)cycloalkyle, et -SO₂-hétéroaryle ;
      - ou, lorsque p représente 0, 1, 2, 3 ou 4, A représente un groupe de formule : 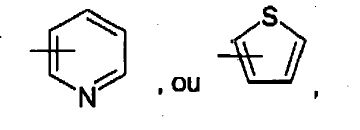 ledit groupe étant éventuellement substitué par un groupe (C₁-C₄)alkyle;
   **a2)** un groupe de formule -(CH₂)ₚ-CO-A
      dans laquelle p représente 1, 2, 3 ou 4,
      - A représente un groupe de formule : 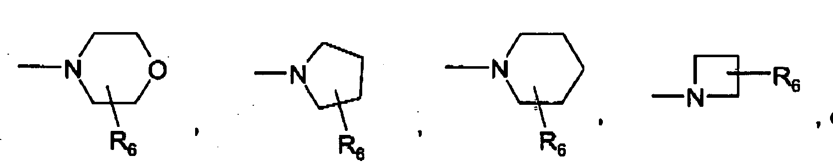 ou -NR₄R₅,
         dans laquelle R₄, R₅ et R₆ sont tels que définis dans ce qui précède;
   **a3)** un groupe de formule -CO(CH₂)ₚ-A
      dans laquelle p représente 0, 1, 2, 3 ou 4
      - lorsque p représente 1, 2, 3 ou 4, A représente un groupe de formule : 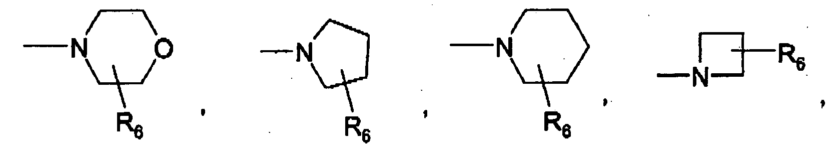 ou -NR₄R₅,
         dans laquelle R₄, R₅ et R₆ sont tels que définis dans ce qui précède;
      - ou, lorsque p représente 0, 1, 2, 3 ou 4, A représente un groupe de formule : 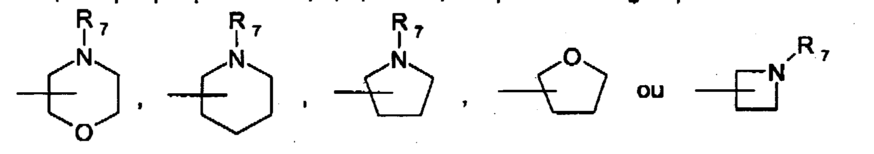 dans laquelle R₇ est tel que défini précédemment ;
      - ou, lorsque p représente 0, 1, 2, 3 ou 4, A représente un groupe de formule: 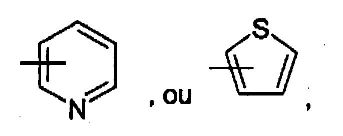 ledit groupe étant éventuellement substitué par un groupe (C₁-C₄)alkyle;
   **a4)** un groupe -B
      dans lequel B représente un groupe de formule : 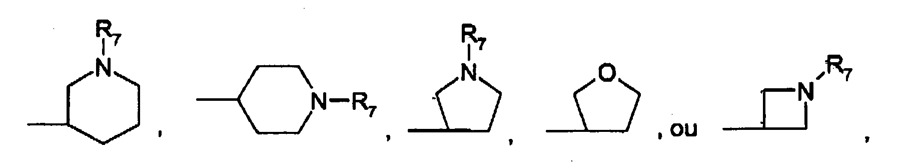 dans laquelle R₇ est tel que défini précédemment;
      - (v) a représente 2 ou 3.

Un halogène préféré est un fluor.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.
Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule (1) peuvent également exister sous forme d'hydrates ou de solvats, à savoir sous forme, d'associations ou de combinaisons avec une ou plusieurs molécules d'eau ou avec un solvant. De tels hydrates et solvats font également partie de l'invention.

Dans le cadre de la présente invention, on entend par :
- C_{t-z} où t et z peuvent prendre les valeurs de 1 à 10, une chaîne carbonée pouvant avoir de t à z atomes de carbone, par exemple C₁₋₃ une chaîne carbonée qui peut avoir de 1 à 3 atomes de carbone ;
- Hal : un atome d'halogène tel qu'un fluor, un chlore, un brome ou un iode ;
- un groupe alkyle : un groupe aliphatique saturé linéaire ou ramifié, éventuellement substitué par un atome d'halogène. A titre d'exemples, on peut citer les groupes méthyle, éthyle, propyle, isopropyle, butyle, isobutyle, tertbutyle, pentyle, 2-fluoroéthyle, etc ;
- un groupe cycloalkyle : un groupe alkyle cyclique. A titre d'exemples, on peut citer les groupes cyclopropyle, cyclobutyle, cyclopentyle, cyclohexyle, etc ;
- un groupe alcoxy : un radical -O-alkyle où le groupe alkyle est tel que précédemment défini ;
- un groupe hétéroaryle : un groupe aryle portant un ou plusieurs hétéroatomes, éventuellement substitué par un autre hétéroatome. A titre d'exemples, on peut citer les groupes pyrazine, pyrimidine, pyridazine, pyridine, triazine, triazole, thiazole; oxazole, pyrazole, imidazole, oxopyridine, etc;
- un groupe perfluoroalkyle : un radical alkyle, tel que défini précédemment, pour lequel tous les atomes de carbone sont substitués par des atomes de fluor.

Parmi les composés objets de l'invention, on peut citer un premier groupe de composés de formule (I.a) suivante : 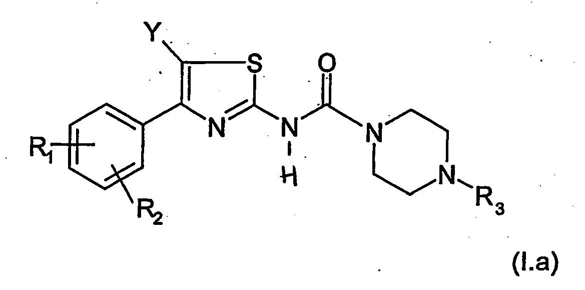 dans laquelle R₁, R₂, R₃ et Y sont tels que définis précédemment.

Des composés de l'invention de formule (I.a) sont ceux dans lesquels R₁ .est en position 2 et R₂ est en position 5 du phényle.

Parmi les composés objets de l'invention, on peut citer un second groupe de composés de formule (I.b) ci-après : 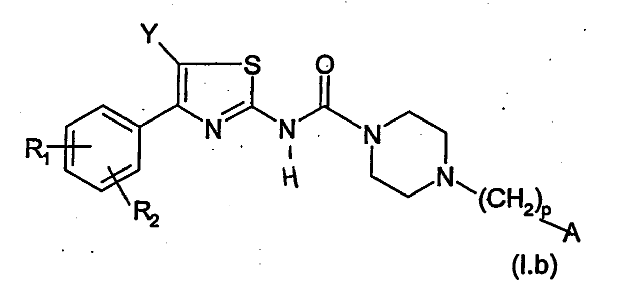 dans laquelle R₁, R₂, Y, p et A sont tels que définis précédemment.

Des composés de l'invention de formule (I.b) sont ceux dans lesquels R₁ est en position 2 et R₂ est en position 5.du phényle.

Parmi les composés objets de l'invention, on peut citer un troisième groupe de composés de formule (I.c) ci-après : 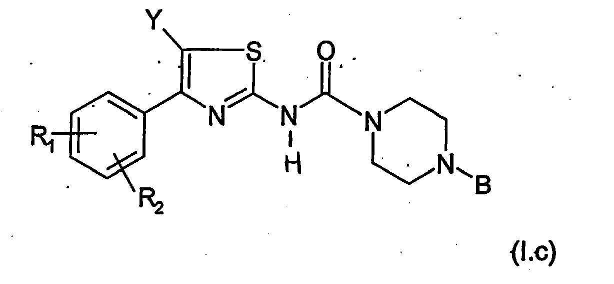 dans laquelle R₁, R₂ , Y et B sont tels que définis précédemment.

Des composés de l'invention de formule (I.c) sont ceux dans lesquels R₁ est en position 2 et R₂ est en position 5 du phényle.

D'autres composés de l'invention sont ceux pour lesquels :
- R₁ représente un groupe -O-(C₁-C₈)alkyle et/ou;
- R₂ représente un groupe (C₁-C₈)alkyle, (C₃C₁₀)cycloalkyle, perfluoro(C₁-C₄)alkyle, -O-(C₁-C₈)alkyle.

Parmi les composés de formule (I) de l'invention, on peut notamment citer les composés suivants :
- (R)-N-[4-(5-cyclohexyl-2-méthoxy-phényl)-thiazol-1-yl]-N'-[4-(1-méthyl-pipéridin-3-ylméthyl)-pipérazin-1-yl]-urée (composé n° 76),
- N-[4-(2-méthoxy-5-propoxy-phényl)-thiazol-2-yl]-N'-[4-(2-morpholin-4-yl-éthyl)-pipérazin-1-yl]-urée (composé n° 1),
- N-[4-(2-méthoxy-5-propyl-phényl)-thiazol-2-yl]-N'-[4-(3-pipéridin-1-yl-propyl)-pipérazin-1-yl]-urée (composé n° 11),
- N-[4-(2-méthoxy-5-propyl-phényl)-thiazol-2-yl]-N'-[4-(2-diméthylamino-éthyl)-pipérazin-1-yl]-urée (composé n° 16),
- [4-(2-méthoxy-5-propyl-phényl)-thiazol-2-yl]-N'-[4-(2-oxo-2-pyrrolidin-1-yl-éthyl)-pipérazin-1-yl]-urée (composé n° 20),
- N-[4-(2-méthoxy-5-propyl-phényl)-thiazol-2-yl]-N'-[4-(2-thiophén-2-yl-éthyl)-pipérazin-1-yl]-urée(compos.é n° 21),
- N-[4-(2-méthoxy-5-propyl-phényl)-thiazol-2-yl]-N'-[4-[2-(tétrahydro-furan-2-yl)-éthyl]-pipérazin-1-yl]-urée (composé n° 22),
- N-[4-(2-méthoxy-5-propy-phényl) thiazol-2-yl]-N'-[4-(2-pyrrolidin-1-yl-acétyl)-pipérazin-1-yl]-urée (composé n° 31),
- N-[4-(2-méthoxy-5-propyl-phényl)-thiazol-2-yl]-N'-[4-[2-(3-éthylamino-pyrrolidin-1-yl)-éthyl]-pipérazin-1-yl]-urée (composé n° 33),
- N-[4-(5-cyclohexyl-2-méthoxy-phényl)-thiazol-2-yl]-N'-[4-(1-méthyl-pipéridin-4-yl)-pipérazin-1-yl]-urée (composé n° 47),
- N-[4-(5-cyclohexy(-2-méthoxy-phényl)-thiazol-2-yl]-N'-[4-(1-benzyl-pipéridin-3-yl)-pipérazin-1-yl]-urée (composé n° 68),
- N-[4-(2-méthoxy 5-propyl-phényl)-thiazol-2-yl]-N'-[4-(2-pyridin-4-yl-éthyl)-pipérazin-1-yl]-urée (composé n° 28),
- N-[4-(2-méthoxy-5-pentafluoroéthyl-phényl)-thiazol-2-yl]-N'-[4-(2-pyrrolidin-1-yl-éthyl)-pipérazin-1-yl]-urée (composé n° 29),
- N-[4-(5-cyctohexy)-2-méthoxy-phényl)-thiazol-2-yl]-N'-[4-(1-)sopropyl-pipéridin-3-yl)-pipérazin-1-yl]-urée (composé n°70),
- N-[4-(5-cyclohexyl-2-méthoxy-phényl)-thiazol-2-yl]-N'-[4-(2-pyrrolidin-1-yl-éthyl)-pipérazin-1-yl]-urée (composé n° 30),
- N-[4-(5-cyclohexyl-2-méthoxy-phényl)-5-fluoro-thiazol-2-yl]-N'-[4-(1-méthyl-pipéridin-4-yl)-pipérazin-1-yl]-urée (composé N° 59),
- N-[4-(5-cyclohexyl-2-méthoxy-phényl)-thiazol-2-yl]-N'-[4-(2-oxo-2-pyrrolidin-1-yl-éthyl)-pipérazin-1-yl]-urée (composé n° 108),
- N-[4-(5-cyclohexyl-2-éthoxy-phényl)-thiazol-2-yl]-N'-[4-(2-oxo-2-pyrrolidin-1-yl-éthyl)-pipérazin-1-yl]-urée (composé n° 109),
- N-[4-(5-cyclohexyl-2-méthoxy-phényl)-thiazol-2-yl]-N'[4-(2-oxo-2-morpholino-4-yl-éthyl)-pipérazin-1-yl]-urée (composé n° 116),
- N-[4-(5-cyclohexyl-2-éthoxy-phényl)-thiazol-2-yl]-N'-[4-(2-oxo-2-morpholino-4-yl-éthyl)-pipérazin-1-yl]-urée (composé n° 110),
- (S) N-[4-(5-cyclohexyl-2-méthoxy-phényl)-thiazol-2-yl]-N'-[4-(1-sulfomethyl-piperidin-3-yl-méthyl)-pipérazin-1-yl]-urée (composé n° 112),
- N-[4-(5-cycohexyl-2-méthoxy-phényl)-thiazol-2-yl]-N'-[4-(1-oxo-1-(1-oxopyridine-2yl-)meth-1-yl-piperidin-3-yl-méthyl)-pipérazin-1-yl]-urée (composé n° 113),

Certains intermédiaires utiles pour la préparation des composés de formule (I) peuvent également servir en tant que produit final de formule (I), ainsi qu'il apparaîtra dans les exemples donnés ci-après.
De façon similaire, certains composés de formule (I) de l'invention peuvent servir en tant qu'intermédiaires utiles pour .la préparation de composés de formule (I) selon l'invention.

Dans ce qui suit, on entend par groupe protecteur, Gp un groupe qui permet, d'une part, de protéger une fonction réactive telle qu'un hydroxy ou une amine pendant une synthèse et, d'autre part, de régénérer la fonction réactive intacte en fin de synthèse. Des exemples de groupes protecteurs ainsi que des méthodes de protection et de déprotection sont donnés dans « Protective Groups in Organic Synthesis », Green et al., 2nd Edition (John Wiley & Sons, Inc., New York).
On entend par groupe partant X, dans ce qui suit, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxy activé tel qu'un mésyle, tosyle, triflate, acétyle, etc. Des exemples de groupes partants ainsi que des références pour leur préparation sont donnés dans « Advances in Organic Chemistry », J. March, 3rd Edition, Wiley Interscience, p. 310-316.

Dans ce qui suit, on entend par précurseur de R₁, R₂ ou R₃, un substituant R'₁, R'₂ ou R'₃ pouvant être transformé en R₁, R₂ et R₃ par une ou plusieurs réactions chimiques.

On entend par groupe Z dans ce qui suit, un groupe partant ou un dérivé fonctionnel de l'acide, tel qu'un chlorure d'acide, un anhydride mixte ou symétrique, ou encore l'acide opportunément activé par exemple avec l'hexafluoro phosphate de benzotriazol-1-yloxytris(diméthylamino) phosphonium (BOP), le O-benzotriazol-1-yl-N,N,N',N'-tétraméthyluronium hexafluorophosphate (HBTU) ou le O-benzotriazol-1-yl-N,N,N',N'-tétraméthyluronium tétrafluoroborate (TBTU).

Lorsqu'un ou plusieurs substituants R'₁, R'₂ et/ou R'₃ représentent un groupement contenant une fonction amine ou hydroxyle, ces fonctions peuvent être protégées intermédiaire ment : une fonction amine peut être protégée par un groupement alcanoyle, benzyle, *tert*-butoxycarbonyle (Boc), benzyloxycarbonyle, ou 9-fluorénylméthokycarbonyle (Fmoc), par exemple ; une fonction hydroxyle peut être protégée sous forme d'éther ou d'ester, par exemple.

Les composés de l'invention peuvent être préparés selon différentes méthodes décrites dans la présente demande de brevet.

Avant d'aborder ces méthodes, il est décrit ci-après les méthodes de préparation des .dérivés aminothiazole de formule (II) et les méthodes de préparation des dérivés amine de formule (III) qui sont utilisés pour la préparation des composés de formule (I) de l'invention.

Les dérivés aminothiazole de formule (II) peuvent être préparés par des méthodes connues telles que celles décrites dans les documents EP 518 731, EP 611 766 et WO 99/15525.
De façon générale, quand Y = H, on fait réagir la thiourée avec une cétone halogénée de formule 1 selon le schéma réactionnel suivant :

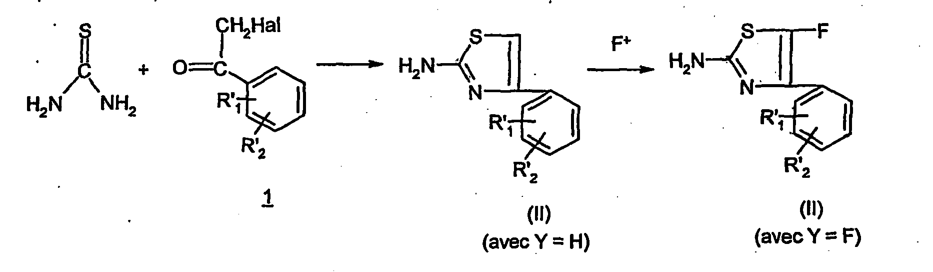

Les substituants R₁, et R'₂ ont les valeurs indiquées plus haut, c'est à dire que R'₁ et R'₂ représentent respectivement R₁ et R₂ tels que définis pour le composé de formule générale (I) ou des groupes précurseurs de R₁ et R₂; Hal représente un atome d'halogène, de préférence te brome, le chlore ou l'iode.
Comme indiqué sur le schéma précédent, les composés de type (II) avec Y = H, R'₁ et R'₂ ayant les valeurs indiquées plus haut, peuvent être transformés en composés de type (11) avec Y = F et R'₁ et R₂ ayant les valeurs indiquées plus haut par réaction avec un agent de fluoration comme par exemple le Selectfluor© dans un solvant comme le DMF ou le DCM à une température variant de 0°C à 50°-C.
Les cétones halogénées de formule 1 peuvent être préparées par des procédés connus de l'homme de l'art. Par exemple, les bromocétones peuvent être obtenues par action du brome, du bromure cuivrique ou du phényltriméthylammoniumtribromure (PTT) sur un dérivé d'acétophénone de formule : 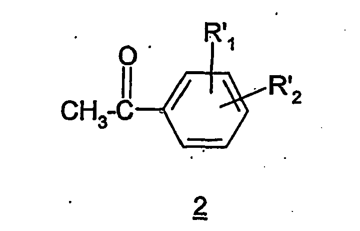 dans laquelle R'₁ et R'₂ ont les valeurs indiquées -ci-dessus, dans un solvant organique tel que l'acétate d'éthyle, un solvant chloré ou leur mélange ou Encore un alcool.
Lorsque le dérivé acétophénone de formule 2 n'est pas disponible , commercialement, on peut le préparer par différentes méthodes :
- une réaction de Friedel-Crafts sur le benzène substitué par R'₁ et R'₂. que l'on fait réagir sur le chlorure d'acétyle ou l'anhydride acétique, en présence d'un acide de Lewis tel que AICl₃ ou TiCl₄, par exemple;
- l'action du chlorure d'acétyle en présence de Palladium sur le benzène substitué par R'₁ et R'₂ après déprotonation du benzène, par exemple par action du butyllithium puis addition du chlorure de zinc ou de l'iodure de manganèse. Ce mode opératoire est utilisable pour préparer un dérivé d'acétophénone de formule 2 dans laquelle R² = R₂ = (C₁-C₄)perfluoroalkyle ;
- un réarrangement de Fries : à partir d'un dérivé d'acétoxybenzène de formule : 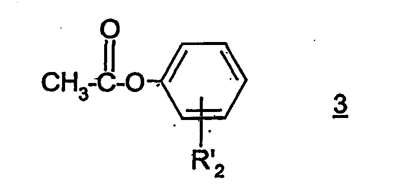 par action d'un acide de Lewis, on obtient un dérivé d'hydroxyacétophénone de formule :

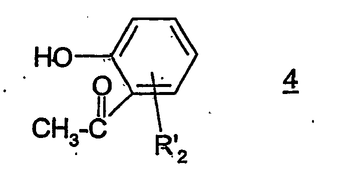

La fonction hydroxyle correspond à un groupement R'₁ que l'on peut transformer dans une étape ultérieure en un groupement -O-W tel que -O-(C₁-C₈)alkyle, trifluorométhoxy, trifluoroéthoxy, allyloxy, (C₃-C₁₀)cycloalkylméthoxy, (C₃-C₁₀)cycloalkyloxy.

La transformation de R'₁ en R₁ peut être effectuée soit sur l'aminothiazole de formule (11), soit sur un composé de formule (I).
Les dérivés du .benzène substitués par R'₁ et R'₂ sont disponibles commercialement ou sont préparés par des méthodes connues de l'homme de l'art.
Par exemple, pour préparer un composé dans lequel R₁ est un groupement -O-W tel que défini ci-dessus, on opère de la façon suivante :

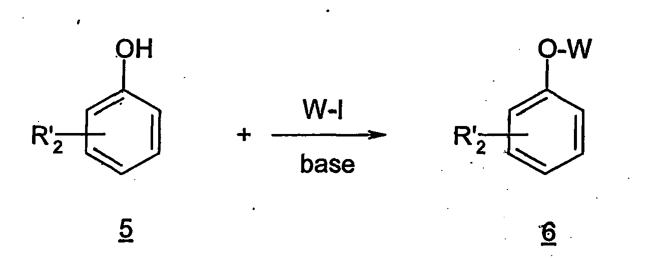

On peut également substituer un dérivé d'halogénobenzène selon le schéma ci-après:

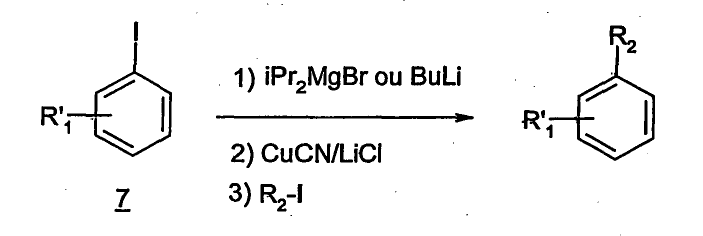

Dans le cas particulier où R₂ représente un (C₁-C₄)perfluoroalkyle, on peut également procéder selon le schéma réactionnel ci-après :

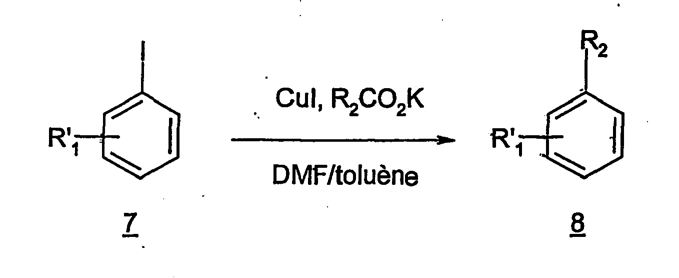

L'invention a également pour objet les composés de formule (II.a) : 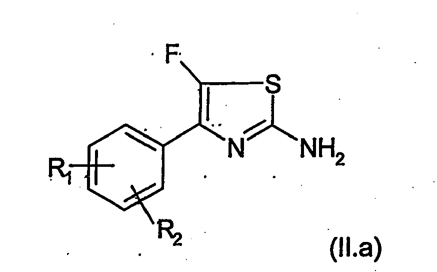 dans laquelle :
- R₁ représente un atome d'halogène, un groupe -O-(C₁-C₈)alkyle, (C₃-C₁₀)cycloalkyl(C₁-C₈)alcoxy ;
- R₂ représente un groupe (C₁-C₈)aLcoxy, (C₁-C₈)alkyle, (C₃-C₁₀)cycloalkyle, trifluoro(C₁-C₄)alkyle, perfluoro(C₁-C₄)alkyle.

Des exemples de préparation de dérivés aminothiazole de formule (II) sont donnés dans ce qui suit.

Les dérivés amine de formule (III) sont connus ou peuvent être préparés selon les méthodes décrites notamment dans le document WO 87/01706 ou selon les méthodes décrites dans ce qui suit.

Dans ce qui suit, le groupe A' représente un groupe précurseur du groupe A ou un groupe A tel que défini précèdemment.

Les composés de formule (III) dans laquelle R'₃ représente un groupe précurseur de R₃ ou un groupe R₃ tel que défini dans ce qui précède et dans laquelle a est tel que défini dans ce qui précède, sont obtenus à partir de composés de formule 9 par déprotection de l'azote de la pipéraziné ou de l'homo-pipérazine protégée selon des méthodes connues de l'homme du métier ou décrites dans la littérature (WO03/104230 et WO03/057145).
A titre d'exemple, on peut procéder comme suit :

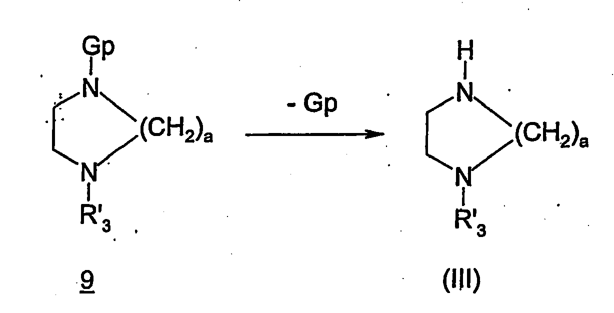

Les composés de formule 9 sont commerciaux ou peuvent être synthétisés à partir de composés commerciaux, selon des méthodes connues de l'homme du métier.

### Préparation 2.1

Les composés de formule 9 dans laquelle R'₃ est un groupe précurseur de R₃ avec R₃ représentant un groupe -(CH₂)ₚ-A, dans lequel A représente : 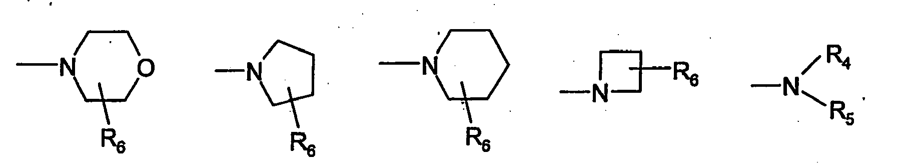 et p est -2, 3 ou 4, -R4, R₅, R₆ étant tels que définis dans ce qui précède, peuvent être préparés par réaction du composé 10 avec A'H en présence d'une base telle que K₂CO₃, la triéthylamine ou le carbonate de césium dans un solvant tel que le THF, l'acétonitrile, le toluène ou le DMF, à des températures allant de 0°C à 150°C, de façon à obtenir le composé 11. Le composé 11 peut ensuite être transformé en un composé de formule 9 par réduction de la fonction amide, par exemple avec le LiAIH₄, l'hydrure de diisobutylaluminium (Dibal); le -BH₃ dans le THF, l'éther ou le toluène à une température comprise entre 0°C et 70°C, pour donner le composé de formule 9 dans lequel R₃ représente -(CH₂)ₚ-A :

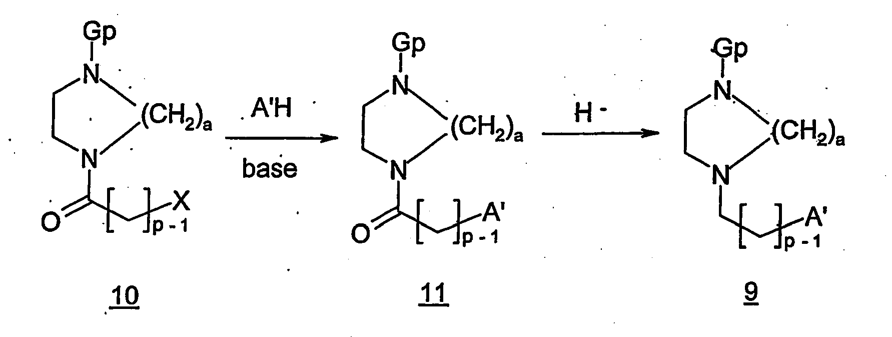

Le composé de formule 10 peut être préparé par réaction d'une pipérazine ou d'une homo-pipérazine mono-protégée avec un réactif de formule 12 ci-après : 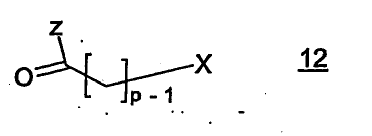 dans laquelle Z représente un groupe partant ou un groupe issu de l'activation d'une fonction acide carboxylique et X représente un groupe partant, dans un solvant tel que le THF, l'acétonitrile, le DMF, ou le dichlorométhane en présence d'une base telle que K₂CO₃, la triéthylamine et, quand Z représente un groupe-OH, d'un réactif activant la fonction acide comme le BOP, le TBTU ou le CDI, selon le schéma ci-après :

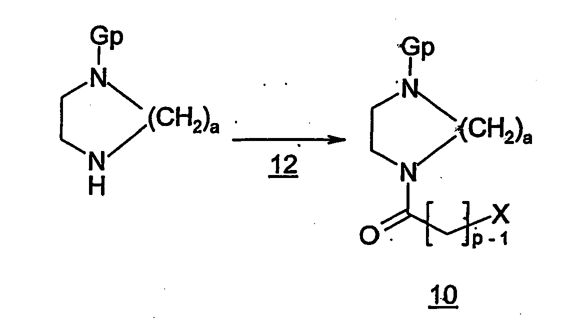

### Préparation 2.2

Les composés de formule 9 dans laquelle R'₃ est un groupe précurseur de R₃ avec R₃ représentant un groupe -(CH₂)p-A, dans lequel A représente : 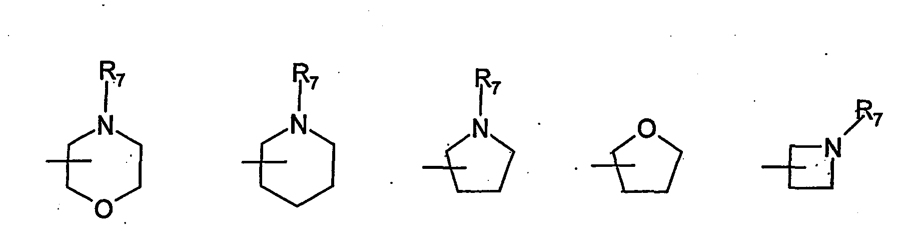 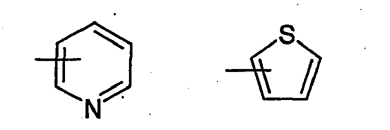 et p représente 1, 2, 3 ou 4, R₇ étant tel que défini dans ce qui précède, peuvent être préparés par réaction du composé 13 avec une pipérazine ou une homopipérazine mono-protégée dans un solvant tel que le THF, l'acétonitrile, le DMF ou le dichlorométhane en présence d'une base telle que K₂CO₃, la triéthylamine et, quand Z représente un groupe -OH, d'un réactif activant la fonction acide comme le BOP, le TBTU ou le CDI, selon le schéma ci-après :

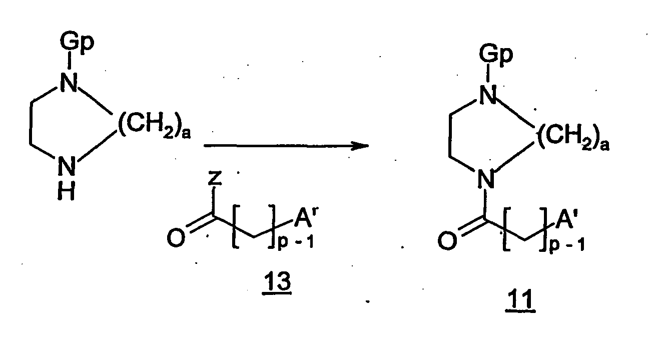

Le composé 11 obtenu peut être ensuite transformé en un composé de formule 9 selon le schéma décrit précédemment.

Le composé 13, quand il n'est pas disponible commercialement, peut être obtenu par homologation de l'acide carboxylique commercial selon des méthodes classiques telle que des réactions de type Amdt-Elstert (Tetrahedron Lett., 1979, 29, 2667; « Advances in Organic Chemistry », J. March, 3rd Edition, Wiley Interscience, p. 1405-1407.), selon le schéma -ci-après

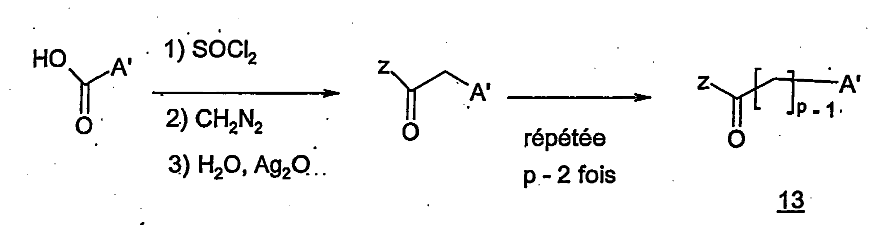

### Préparation 2.3

Les composés de formule 9 dans laquelle R'₃ représente un groupe précurseur du groupe R₃ avec R₃ représentant -(CH₂)ₚCO-A tel que défini dans ce qui précède peuvent être obtenus à partir des composés de formule 14 dans laquelle Z est tel que défini précédemment, par réaction avec A'H en présence d'une base telle que K₂CO₃, la triéthylamine ou le carbonate de césium et, quand Z représente un groupe -OH, d'un réactif activant la fonction acide comme le BOP, le TBTU ou le CDI dans un solvant comme par exemple le THF, l'acétonitrile ou le DMF à des températures allant de 0°C à 150°C, selon le schéma ci-après :

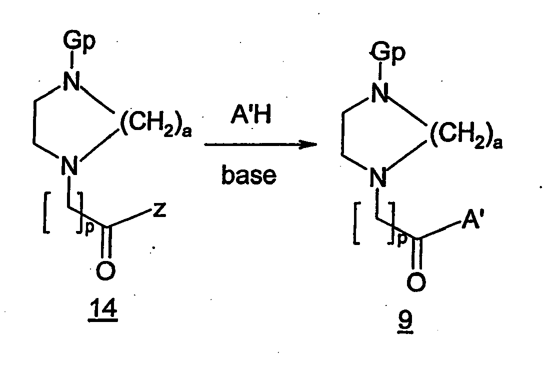

Les composés de formule 14, lorsqu'ils ne sont pas disponibles commercialement, peuvent être obtenus à partir d'une pipérazine ou d'une homo-pipérazine mono-protégée et du réactif 15 dans lequel Z est tel que défini précédemment, par acylation ou couplage de type peptidique en présence d'une base telle que K₂CO₃, la triéthylamine ou le carbonate de césium ou d'un réactif de couplage comme le BOP, le TBTU ou le CDI, dans un solvant comme par exemple le THF, l'acétonitrile ou le DMF à des températures allant de 0°C à 150°C, selon le schéma ci-après :

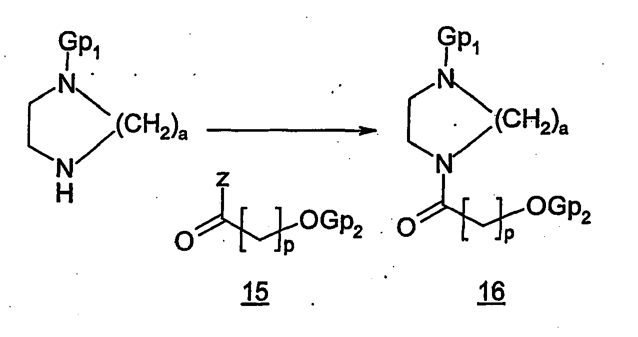

Le composé 16 est ensuite transformé par réduction de la fonction amide avec par exemple le LiAlH₄ dans le THF ou l'ether éthylique à une température comprise entre 0 et 50°C. L'intermédiaire obtenu est déprotégé puis oxydé en acide carboxylique 17 par exemple avec le CrO₃ ou avec d'autres réactifs selon les méthodes décrites dans « Advances in Organic Chemistry », J. March, 3rd Edition, Wiley Interscience, p. 1537-1539, selon le schéma ci-après :

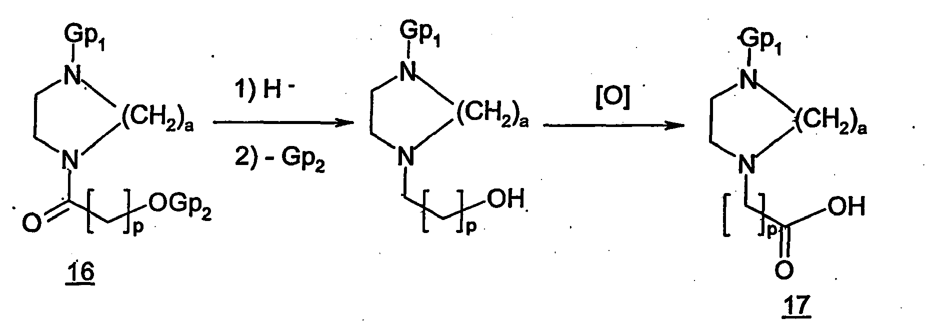

Le composé 17 est ensuite éventuellement transformé pour donner un composé de formule 14 ou utilisé sous sa forme acide (Z = OH).

Alternativement, les composés de formule 17 peuvent être préparés par réaction d'une pipérazine ou d'une homo-pipérazine mono-protégée avec un réactif 18 de formule: 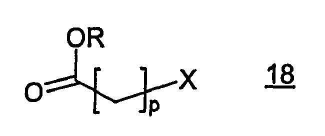 dans laquelle X est tel que défini précédemment et R représente un groupe (C₁-C₄)alkyle, par alkylation de l'azote en présence d'une base telle que K₂CO₃; la triéthylamine ou le carbonate de césium dans un solvant tel que THF, l'acétonitrile, le toluène ou le DMF à des températures allant de 25 à 150 °C, selon le schéma ci-après :

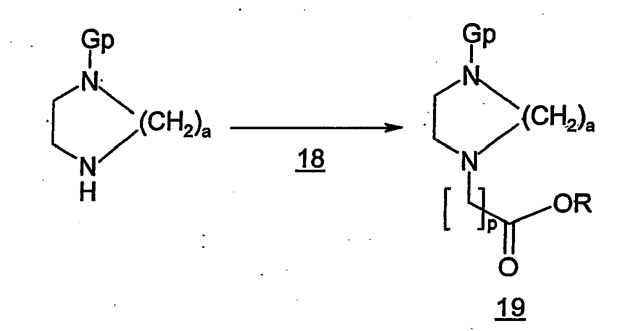

Le composé 19 obtenu est ensuite transformé en acide de formule 17 par saponification ou hydrolyse acide ou toute autre méthode connue de l'homme du métier.

### Préparation 2.4

Les composés de formule 9 dans laquelle R'₃ représente un groupe précurseur du groupe R₃ avec R₃ représentant un groupe -CO(CH₂)ₚ-A dans lequel A représente : 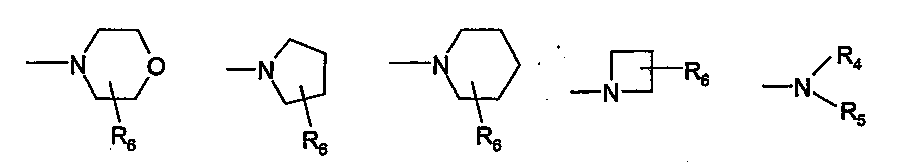 et p représentant 1, 2, 3, 4 et R₄, R₅, R₆ étant tels que définis dans ce qui précède peuvent être préparés en utilisant la méthode décrite dans la préparation 2.1 ci-dessus.

### Préparation 2.5

Les composés de formule 9 dans laquelle R'₃ représente un groupe précurseur du groupe R₃ avec R₃ représentant un groupe -CO(CH₂)ₚ-A dans lequel A représente : 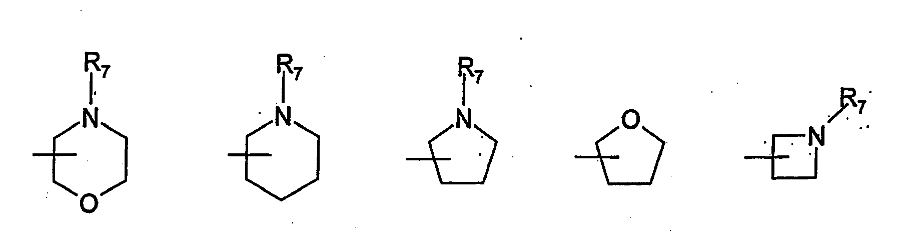 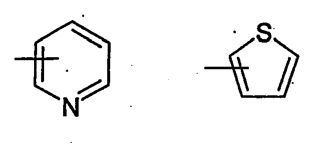 et p représentant 0, 1, 2, 3, 4 et R₇ étant tel que défini dans ce qui précède peuvent être préparés en utilisant la méthode décrite dans la préparation 2.1 ci-dessus.

### Préparation 2.6

Les composés de formule 9 dans laquelle R'₃ représente un groupe précurseur du groupe R₃ avec R₃ représentant un groupe -B peuvent être préparés par réaction d'une pipérazine ou d'une homo-pipérazine mono-protégée et d'une cétone B' précurseur de B, par réaction d'amination réductrice en présence d'un agent réducteur tel que NaHB(OAc)₃, NaBH₃CN dans un solvant tel que le 1,2-dichloroéthane, le dichlorométhane, le THF à des températures allant de 0°C à 70°C (Synth. Commun., 1998, 28 (10), 1897-1905, J. Org. Chem., 1992, 57 (11), 3218-3225, J. Org. Chem, 1996, 61, 3849-3862, Tetrahedron Lett., 1990, 31, 5595-5598.), selon le schéma ci-après :

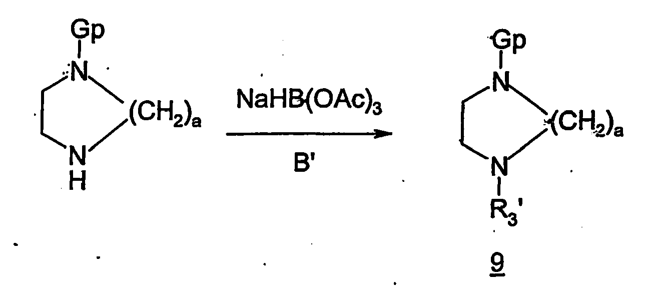

Les cétones B' utilisées sont commerciales ou peuvent être synthétisées selon la méthode décrite dans J. Org. Chem., 1989, 54,1249-1256.

Les composés de formule (I) de l'invention peuvent être préparés selon le schéma général 1 ci-après.

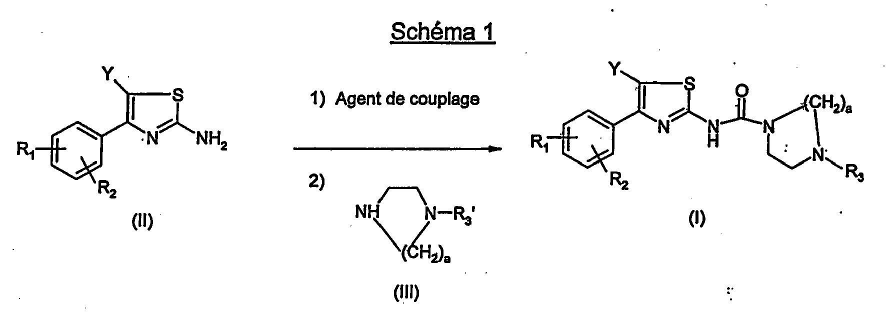

Selon le schéma 1, les composés de l'invention sont obtenus par couplage du dérivé aminothiazole de formule (11) dans laquelle R₁, R₂, Y sont tels que définis dans ce qui précède, avec un dérivé amine de formule (III) dans laquelle R'₃ représente un groupe précurseur de R₃ ou un groupe R₃ tel que défini dans ce qui précède et a est tel que défini dans ce qui précède.
Selon le schéma 1, le dérivé aminothiazole de formule (II) est mis en présence d'un agent de couplage pendant une durée de 2 à 16 heures, puis avec le dérivé amine de formule (III) pendant une durée de 0,5 à 4 heures.
L'agent de couplage peut être choisi parmi ceux connus de l'homme du métier, par exemple le phosgène, le di-(N-succinimidyl)carbonate, le 1,1'-carbonyldiimidazole, selon les méthodes décrites dans « Encyclopedia of Reagents for Organic Synthesis», L. A. Paquette, volume 2, p 1006; volume 4, p 2304; volume 6, p 4107.
La réaction peut être réalisée dans différents solvants, par exemple le dichlorométhane, le diméthylformamide, le toluène, en présence d'une base telle que la triéthylamine, K₂CO₃, à une température variant de 0°C à 100°C.

Les composé de l'invention de formule (I) dans laquelle R₃ représente un groupe -CO(CH₂)ₚ-A (ou -CO(CH₂)ₚ-A') dans lequel A et p sont tels que définis dans ce qui précède et A' représente un groupe précurseur de A peuvent également être préparés selon le schéma 2 ci-après.

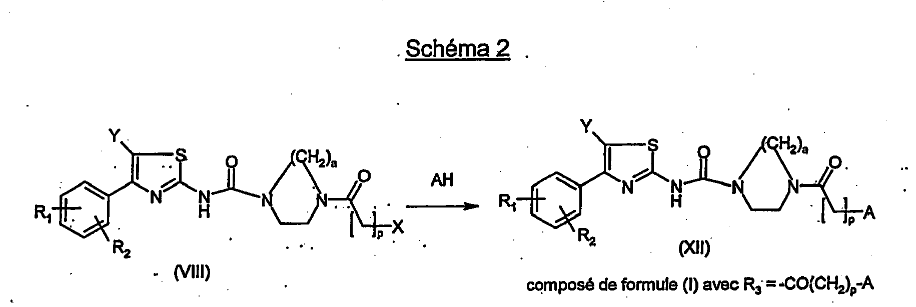

Selon le schéma 2, le dérivé aminothiazole de formule (VIII) dans laquelle R₁, R₂, Y, a et p sont tels que définis dans ce qui précède et X représente un groupe partant est mis à réagir avec un groupe A'H précurseur du groupe A ou un groupe AH tel que défini dans ce qui précède pour donner le composé de formule (XII) (composé de formule (I) dans laquelle R₃ représente un groupe -CO(CH₂)ₚ-A).
La réaction est réalisée dans un solvant tel que le tétrahydrofurane, le diméthylformamide en présence d'une base telle que la triéthylamine ou K₂CO₃, à des températures variant de la température ambiante à 150°C, pendant une durée de 1 à 24 heures.

Les composés de l'invention de formule (I) dans laquelle R₃ représente un groupe -(CH₂)p-A peuvent être préparés de façon connue, à partir du composé de formule (XII) tel que défini ci-dessus, directement par réduction de la fonction carbonyle avec un agent réducteur tel que le Red-Al, LiAlH₄ selon le schéma 3 ci-après :

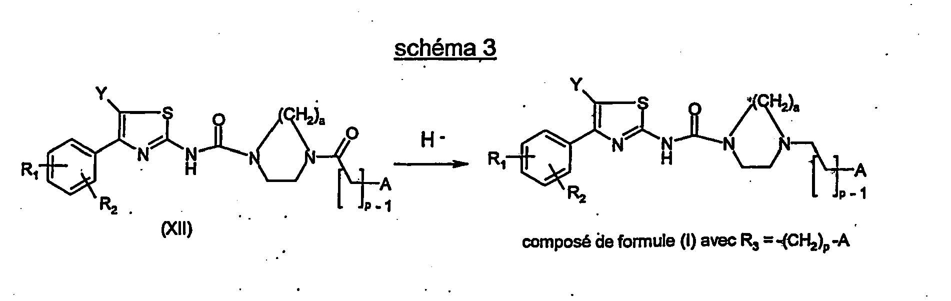

Alternativement, les composés de formule (I) peuvent être préparés à partir du composé de formule (XII) dans lequel le groupe A est protégé préalablement à la réaction de réduction, notamment lorsque le groupe A comporte des fonctions incompatibles avec le type d'agent réducteur utilisé. Après réduction, un composé de formule (I) est ensuite obtenu par déprotection du groupe A et une éventuelle fonctionnalisation du groupe A.

Les composés de formule (VIII) peuvent être préparés selon le schéma 4 ci-après.

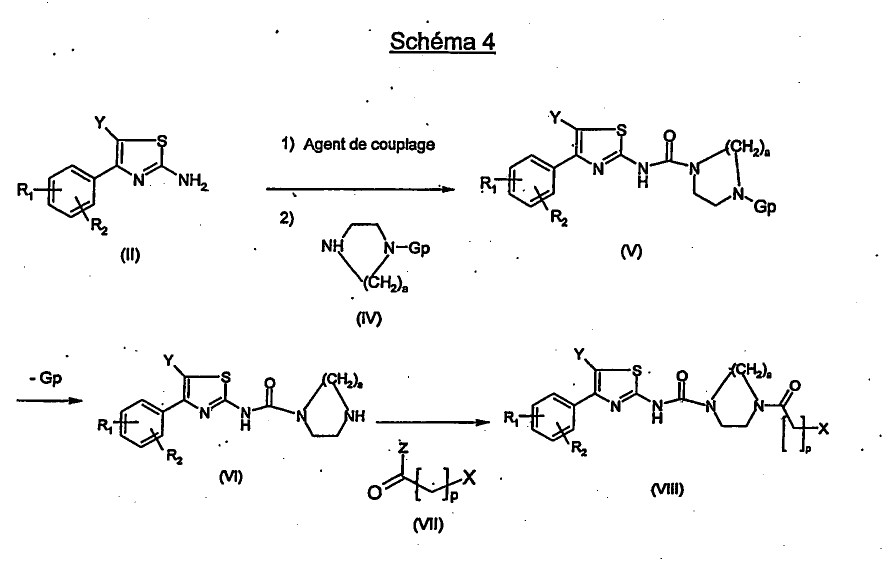

Selon le schéma 4, le dérivé aminothiazole de formule (H) tel que défini dans ce qui précède est couplé à un dérivé amine de formule (IV) dans laquelle Gp représente un groupe protecteur, par exemple un groupe benzyle ou Boc, et a est tel que défini dans ce qui précède, pour donner le composé de formule (V).
La réaction est conduite dans les mêmes conditions que celles décrites ci-dessus pour le schéma 1.
Le composé de formule (V) est ensuite déprotégé pour conduire au composé dé formule (VI), selon les méthodes connues de l'homme du métier, qui est mis à réagir avec le composé de formule (VII) dans laquelle Z représente un groupe partant ou un groupe issu de l'activation d'une fonction acide carboxylique et X un groupe partant pour donner le composé de formule (VIII) dans laquelle R₁, R₂, Y, X et a sont tels que définis dans ce qui précède.

Alternativement, les composés de formule (VIII) peuvent être préparés selon le schéma 5 ci-après.

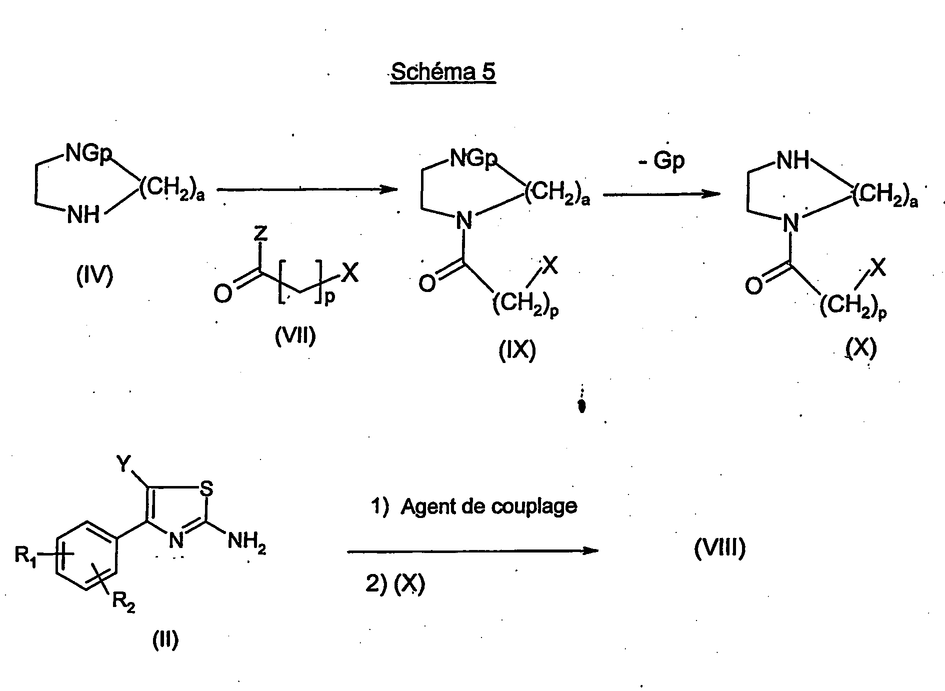

Selon le schéma 5, le composé de formule (VII) dans lequel X est tel que défini précédemment et Z représente un groupe partant ou un groupe issu de l'activation d'une fonction acide carboxylique, peut être couplé au composé de formule (IV) par acylation ou couplage de type peptidique en présence d'une base telle que K₂CO₃, la triéthylamine ou le carbonate de césium ou d'un réactif de couplage comme le BOP, le TBTU ou le CDI, dans un solvant comme par exemple le THF, l'acétonitrile ou le DMF à des températures allant de 0°C à 150°C. Le composé (IX) est ainsi obtenu. Le composé (X), obtenu par déprotection du composé (IX), est ensuite couplé avec un composé aminothiazole de formule (II) dans des conditions identiques à celles du schéma 1.

Dans les schémas généraux de synthèse, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les exemptes qui suivent décrivent" la préparation de composés conformes, à l'invention. Ces exemples ne sont pas limitatifs et ne font qu'illustrer la présente invention. Les numéros des composés exemplifiés renvoient à ceux donnés dans le tableau II, qui illustre les structures chimiques et les propriétés physiques de quelques composés selon l'invention.

Dans les préparations et exemples qui suivent :
- CyHex = un groupe cyclohexyle ;
- TA = température ambiante ;
- DCM = dichlorométhane
- DSC = di-(N-succinimidyl)carbonate
- DIPEA = diisopropyléthylamine
- THF = tétrahydrofurane
- BOP = benzotriazole-1-yle-oxy-tris(diméthylamino)-phosphoniumhéxafluorophosphate
- PF = point de fusion
- CDI = 1,1'-carbonyl-diimidazole
- DMF = diméthylformamide
- DCE = dichloroéthane
- TFA = trifluoroacétic acid
- Red-al^{®} = hydrure de sodium bis(2-metoxyethoxy)aluminium
- TBME = *tert-*butyl-methyl-ether
- TBTU = 2-(1H-Benzotriazole-1-yl)-1,1,3,3-tetramethyluroniumtetrafluoroborate

### Préparation de dérivés aminothiazole de formule (II)

Composés de formule (II) dans laquelle :
Y représente - H (préparations 1.1 à 1.25),
R₁ et R₂ sont en position 2 et 5 respectivement du phényle.

### Préparation 1.1

### 4-(2-Méthoxy-5-propoxyphényl)-1,3-thiazol-2-amine.

### A) 1-(2-Hydroxy-5-propoxyphényl)éthanone.

Dans un ballon de 500 mL sont placés 10 g de 2,5-dihydroxyacétophénone en suspension dans 100 mL d'acétone, 9,14 g de K₂CO₃ anhydre sont ajoutés suivis par 12,4 g d'iodure de propyle. Le milieu réactionnel est chauffé à reflux pendant 30 heures. Après retour à température ambiante, le milieu est filtré sur Célite^{®} puis concentré. L'huile brune obtenue est reprise dans l'AcOEt, filtrée, lavée à l'eau, avec une solution d'HCl 2M, puis avec une solution raturée de NaCl. La phase organique est évaporée pour donner une pâte noire. La pâte est reprise dans le chloroforme et filtrée. Le milieu est concentré pour donner 11,4 g d'un solide noir. Ce dernier est repris dans l'éthanol absolu. La solution est placée 10 minutes au congélateur, un solide précipite, il est collecté par filtration. Le filtrat est concentré, repris dans l'éthanol, refroidi au congélateur puis filtré à nouveau. Cette opération est répétée 4 fois pour donner 8,35g du composé attendu sous forme d'une poudre.

### B) 1-(2-Méthoxy-5-propoxyphényl)éthanone.

A une solution de 35 g du solide précédent dans 350 mL de DMF, on ajoute 49,8 g de K₂CO₃ puis 22,4 mL d'iodure de méthyle. Le milieu réactionnel est chauffé pendant 12 heures à 60°C. Après retour à température ambiante, le milieu est filtré sur Célite^{®}, dilué dans l'éther et lavé avec une solution d'HCl 2M. La phase aqueuse est extraite 2 fois dans l'éther. Les phases organiques rassemblées sont lavées avec une solution de soude diluée, puis lavées à l'eau 2 fois et avec une solution saturée de NaCl. La phase organique est séchée sur MgSO₄, puis évaporée pour donner 35,55g d'une huile brune. L'huile est distillée sous pression réduite à 115°C, pour donner 32,8 g du composé attendu sous forme d'une huile.

### C) 2-Bromo-1-(2-méthoxy-5-propoxyphényl)éthanone.

A une solution de 16,4 g de l'huile obtenue à l'étape précédente dans 100 mL de méthanol, on ajoute au goutte à goutte 4,8 mL de brome. Le milieu est agité 30 minutes à température ambiante, puis évaporé. L'huile obtenue est reprise dans le dichlorométhane, lavée 3 fois dans l'eau, puis séchée sur MgSO₄ et évaporée pour donner 24,5 g d'une huile brune.

### D) 4-(2-Méthoxy-5-propoxyphényl)-1,3-thiazol-2-amine.

A une solution de 42 g de la bromocétone préparée à l'étape précédente dans 200 mL d'éthanol, sont ajoutés 24,5 g de thiourée. Le milieu est porté à reflux pendant 1 heure 30 minutes. Le milieu est alors placé au réfrigérateur pendant 12 heures, .puis filtré. Le solide ainsi collecté est rincé avec un peu d'éthanol froid, puis à l'éther. On récupère 25 g de bromhydrate.
Le solide est mis en suspension dans un mélange eau/dichlorométhane et on effectue un retour à la base par addition de soude. La phase aqueuse est extraite 2 fois dans le dichlorométhane. Les phases organiques rassemblées sont séchées sur MgSO₄, puis évaporées; L'huile obtenue est chromatographiée sur gel de silice pour donner 12 g du produit attendu sous forme de poudre. PF = 76°C

### Préparation 1.2

### 4-(5-Butyl-2-méthoxyphényl)-1,3-thiazol-2-amine

### A) 4-Butylphényl acétate

Une solution de 10 g de 4-n-butylphénol, 10 mL d'Ac₂O et 8 mL de pyridine est agitée à reflux dans 10 mL de dichlorométhane. Après 2 heures, le milieu est refroidi à température ambiante, dilué dans le dichlorométhane, lavé à l'eau, lavé avec une solution d'HCl 1M, lavé dans une solution de CuSO₄ saturée, lavé à l'eau et séché sur MgSO₄. Après évaporation, on récupère 10,8 g du composé attendu sous forme d'une huile.

### B) 1-(5-Butyl-2-hydroxyphényl)éthanone

A 5 g de l'huile obtenue à l'étape précédente dans un ballon de 100 mL, on ajoute en plusieurs fois 3,22 g d'AlCl₃. Le milieu est chauffé à 130°C pendant 1 heure. Après retour à température ambiante, une solution d'eau glacée acidifiée par du HCl 35 % est coulée sur le brut réactionnel. Le milieu est placé dans un bain à ultrasons. On additionne de l'AcOEt pour obtenir, après 15 minutes, la solubilisation du milieu. La phase aqueuse est extraite 3 fois dans l'AcOEt, les phases organiques sont lavées à l'eau; puis avec une solution saturée de NaCl. Après séchage sur MgSO₄ et évaporation, on récupère 4,5 g d'une huile jaune.

### C) 1-(5-Butyl-2-méthoxyphényl)éthanone

A une solution d'1 g de l'huile obtenue à l'étape précédente dans 10 mL de DMF, on ajoute 1,44 g de K₂CO₃ puis 0,648 mL d'iodure de méthyle. Le milieu est chauffé à 60°C pendant une huit. Après retour à température ambiante, le milieu est filtré sur Célite^{®}, dilué dans l'éther et lavé avec une solution d'HCl 2M. La phase aqueuse est extraite 2 fois dans l'éther. Les phases organiques rassemblées sont lavées avec une solution de soude diluée, puis lavées à l'eau deux fois et avec une solution saturée de NaCl. La phase organique est séchée sur MgSO₄, puis évaporée pour donner 1,27 g d'une huile brune. L'huile est purifiée par chromatographie pour donner 0,66 g du composé attendu.

### D) 4-(5-Butyl-2-méthoxyphényl)-1,3-thiazol-2-amine

A une solution de 0,66 g du produit de l'étape précédente dans 10 mL de méthanol, on additionne 0,19 mL de brome. Le milieu est agité pendant 10 minutes, puis évaporé et repris dans le dichlorométhane. La phase organique est lavée 3 fois à l'eau, puis séchée sur MgSO₄. On récupère 0,79 g du produit attendu après évaporation. Ce composé est dissout dans 5 mL d'éthanol en présence de 0,46 g de thiourée et le milieu est porté à reflux pendant 2 heures 30 minutes. Un solide précipite lors du retour à température ambiante. Le solide ainsi collecté est rincé avec un peu d'éthanol froid, puis à l'éther. On récupère ainsi 0,6 g du bromhydrate.
Le solide est mis en suspension dans un mélange eau/dichlorométhane et on effectue un retour à la base par addition de soude. La phase aqueuse est extraite 2 fois dans le dichlorométhane. Les phases organiques rassemblées sont séchées sur MgSO₄, puis évaporées pour donner 0,34 g d'une huile jaune qui cristallise lentement. Les eaux mères sont évaporées, puis agitées dans un mélange eau/dichlorométhane et on effectue un retour à la base par addition de soude. La phase aqueuse est extraite 2 fois dans le dichlorométhane. Les phases organiques rassemblées sont séchées sur MgSO₄, puis évaporées. L'huile obtenue est chromatographiée sur gel de silice pour donner 0,18 g du composé attendu.
PF = 48°C.

### Préparation 1.22

### 4-(5-Pentafluoroethyl-2-methoxy-phenyl)-1,3-thiazola-amine

### A) 1-Methoxy-4-pentafluoroethyl-benzene

Sous atmosphère inerte, dans un tricol de 500 ml équipé d'un Dean-Starck et d'un réfrigérant, on introduit 8,3 g de pentafluoropropionate de potassium et 9,8 g de Cul. On ajoute 90 ml de DMF et 110 ml de toluène. Le milieu est chauffé à 140°C sous azote, 80 ml de toluène sont distillés. Le milieu est alors refroidi à TA puis dé-oxygéné avec un bullage à l'azote. On ajoute ensuite 6 g de iodoanisole puis on chauffe à 155°C pendant 20 h. Après retour à TA, le milieu est dilué dans 200 ml d'un mélange eau / éther éthylique. Le milieu est alors filtré sur Célite®. La phase organique est lavée 3 fois à l'eau, séchée sur MgSO₄ puis évaporée pour donner 4,3 g d'une huile marron.

### B) 1-(2-Methoxy-5-pentafluoroethyl-phenyl)-ethanone

A une solution de 3,5 g de 1-Methoxy-4-pentafluoroethyl-benzene dans 50 ml de THF anhydre, on additionne, à -70°C, 7,4 ml de BuLi à 2,5 M dans l'hexane. Le milieu est agité 30 min à -70°C puis 45 min à 0°C. On additionne alors 15,5 ml d'une solution de chlorure de zinc 1 M dans l'éther. Après 10 min d'agitation à 0°C, 1,33 ml de chlorure d'acétyle est ajouté. Le milieu est alors dé-oxygéné à l'azote et 332 mg de palladium benzyl(chloro)-bis(triphenylphosphine) dans 5 ml de THF anhydre sont introduits. Le milieu .est agité 2 h 30 à 0°C puis 72 heures à TA. Le milieu est coulé sur une solution d'HCl 2,5 M puis extrait dans l'éther. La phase organique est lavée au NaHCO₃ à 5 % dans l'eau, à l'eau puis avec une solution saturée de NaCl. Après séchage sur MgSO₄ et évaporation, le brut est purifié par flash-chromatographie sur silice pour donner 2,25 g d'un solide blanc.
PF = 47°C.

### C) 4-(2-Methoxy-5-pentafluoroethyl-phenyl)-thiazol-2-ylamine

A une solution de 2,25 g du produit obtenu à l'étape précédente dans 10 ml de méthanol, on additionne 0,5 ml de brome en solution dans 8 ml de méthanol. Le milieu est agité pendant 10 min puis évaporé et repris dans le dichlorométhane. La phase organique est lavée 3 fois à l'eau puis séchée sur MgSO₄. On récupère 2,63 g du produit bromé après évaporation. Ce composé est dissout dans 15 ml de méthanol en présence de 1,25 g de thiourée et le milieu est porté à reflux pendant 2 h. Un solide précipite lors du retour à TA. Le solide ainsi collecté est rincé à l'éther éthylique. Le solide est mis en suspension dans un mélange eau / dichlorométhane et on effectue un retour à la base par addition de soude. La phase aqueuse est extraite 2 fois dans le dichlorométhane. Les phases organiques rassemblées sont séchées sur MgSO₄ puis évaporées pour donner 1,63 g d'un solide jaune.
PF =125°C

### Préparation 1.3

### 4-(5-Cyclohexyl-2-méthoxyphényl)-1,3-thiazol:2-amine

A) A une solution de 5 g de 4-cyclohe,xylphénol dans 60 mL de DMF, on ajoute 7,84 g.de K₂CO₃, puis 3,53 mL d'iodure de méthyle. Le milieu est chauffé à 60°C. pendant une nuit. Après retour à température ambiante, le milieu est filtré sur Célite®, puis dilué dans l'éther et hydrolysé à l'eau La phase aqueuse est acidifiée, puis extraite dans 3 fois 50 mL d'éther. Les phases organiques rassemblées sont lavées avec une solution de soude diluée, puis lavées à l'eau 2. fois et avec une solution saturée de NaCl. La phase organique est séchée sur MgSO₄, puis évaporée pour donner 4,31 g du composé attendu sous forme d'un solide.
PF = 67°C.

### B)1-(5-Cyclohexyl-2-méthoXyphényl)éthanone

Une suspension de 5,6 g d'AlCl₃ dans 40 mL de dichlorométhane est refroidie à -10°C. On ajoute 3 mL d'AcCl et 4 g du composé de l'étape précédente. Le milieu est agité 1 heure à -10°C, puis versé dans un bécher contenant de la glace mélangée à de l'HCl à 35%. Après décantation, les phases organiques rassemblées sont séchées sur MgSO₄, puis évaporées pour donner 4,54 g du produit attendu.

### C) 4-(5-Cyclohexyl-2-méthoxyphényl)-1,3-thiazol-2-amine

A une solution de 4,5 g du produit de l'étape précédente dans 25 mL de méthanol, on ajoute, au goutte à goutte 1,16 mL de brome. Le milieu est agité 30 minutes à température ambiante, puis devient très visqueux. 5 mL de méthanol sont rajoutés, suivis de 3,23 g de thiourée. Le milieu est chauffé à reflux pendant 2 heures. Après retour à température ambiante, un solide précipite. Le solide est collecté, puis rincé avec un peu de méthanol froid. Le solide est mis en suspension dans un mélange eau/dichlorométhane et on effectue un retour à la base par addition de soude. La phase aqueuse est extraite 2 fois dans le dichlorométhane. Les phases organiques rassemblées sont séchées sur MgSO₄, puis évaporées pour donner 3,33 g du composé attendu sous forme d'un solide.
PF =113°C.

### Préparation 1.4

### 4-(2-Méthoxy-5-propyl-phenyl)-1,3-thiazol-2-amine

### A) 1-(2-methoxy-5-propyl-phenyl)-ethanone

Une suspension de 10,6 g d'AlCl₃ dans 150 mL de dichlorométhane est refroidie à -10°C. On ajoute 5,7 mL d'AcCl et 6 g de 4-propyl anisole. Le milieu est agité 30 min à -10 °C puis versé dans un bécher contenant de la glace mélangée à de l'HCl à 35%. Après décantation, la phase aqueuse est extraite 3 fois dans le dichlorométhane, les phases organiques rassemblées sont lavées à l'eau, avec une solution saturée de NaCl, séchées sur MgSO₄ puis évaporées pour donner 7,86 g d'une huile brune (quant.).

### B) 2-Bromo-1-(2-methoxy-5-propyl-phenyl)-ethanone

A une solution de 7,86 g du composé obtenu à l'étape précédente dans 80 mL de méthanol, on ajoute au goutte à goutte 2,46 mL de brome dilué dans 40 mL de méthanol. Le milieu est agité 30 min à TA puis évaporé. L'huile obtenue est reprise dans le dichlorométhane, lavée 3 fois dans l'eau puis séchée sur MgSO₄ et évaporée pour donner 11,25 g (quant.) d'une huile jaune.

### C) 4-(2-Méthoxy-5-propyl-phenyl)-1,3-thiazol-2-amine

A une solution de 8 g du composé obtenu à l'étape précédente dans 60 mL d'éthanol sont ajoutés 4,94 g de thiourée. Le milieu est porté au reflux pendant. 1h30 min. Le milieu est alors placé au réfrigérateur pendant 12 h puis filtré. Le solide ainsi collecté est rincé avec un peu d'éthanol froid puis à l'éther. La procédure est répétée une deuxième fois. Le solide est mis en suspension dans un mélange eau/dichlorométhane et on effectue un retour à la base par addition de soude. La phase aqueuse est extraite 2 fois dans le dichlorométhane. Les phases organiques rassemblées sont séchées sur MgSO₄ puis évaporées pour donner 4,89 g d'une huile brune qui cristallise lentement (67%).
Les eaux mères sont évaporées puis agitées dans un mélange eau/dichlorométhane et on effectue un retour à la base par addition de soude. La phase aqueuse est extraite 2 fois dans le dichlorométhane. Les phases organiques rassemblées sont séchées' sur MgSO₄ puis évaporées. L'huile obtenue est chromatographiée sur gel de silice pour donner 580 mg du produit attendu.
Rdt (total) : 75%
PF = 84°C

En opérant selon les modes opératoires ci-dessus, on prépare les composés de formule (II) décrits dans le tableau I ci-après.

**Tableau I**

| | | | | |
|---|---|---|---|---|
| 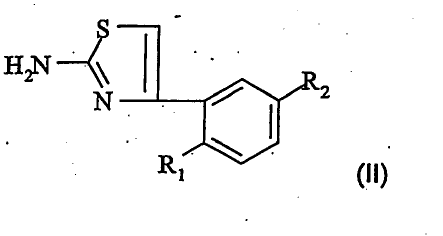 | | | | |

| Préparations n° | R₁ | R₂ | Sel | PF (°C) M |
|---|---|---|---|---|
| 1.1 | -OMe | -OPr | - | PF = 76°C |
| 1.2 | -OMe | -nBu | - | PF = 48°C |
| 1.2 bis | -OMe | -nBu | HBr | PF =186°C |
| 1.3 | -OMe | 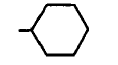 | - | PF =113°C |
| 1.4 | -OMe | -nPr | - | PF = 84°C |
| 1.5 | -OEt | -Et | - | PF = 83°C |
| 1.6 | -OMe | | - | PF = 100°C |
| 1.7 | -OEt | 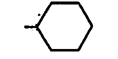 | - | PF = 110°C - |
| 1.8 | -OMe | 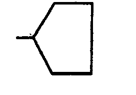 | - | PF=110°C |
| 1:9 | -OEt | -nBu | - | PF = 85°C |
| 1.10 | -OMe | CF₃ | - | PF =144°C |
| 1.11 | -OMe | -iPr | - | PF =109°C |
| 1.12 | -OMe | -Me | - | PF = 121 °C |
| 1.13 | 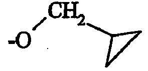 | -nBu | - | PF = 59°C |
| 1.14 | -OMe | 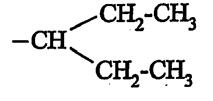 | - | PF = 91-93°C |
| 1.16 | -Cl | CF₃ | - | PF =110°C |
| 1.17 | -OEt | Me | - | PF = 124°C |
| 1.18 | -OMe | CH(nPr)₂ | HCl | MH⁺ = 305,4 t=7,61 |
| 1.19 | -OnPr | -nBu | - | PF = 63°C |
| 1.20 | -OMe | -nHex | - | PF = 43°C |
| 1.21 | -OEt | -nHex | - | PF = 75°C |
| 1.22 | -OMe | CF₃CF₂ | - | PF = 125°C |
| 1.23 | -OEt | CF₃CF₂ | - | MH⁺ = 338 t = 7,88 |
| 1.24 | -OEt | -nPr | - | PF = 87°C |
| 1.25 | -OEt | cyclopentyle | - | PF =128°C |

### Préparation de dérivés aminothiazole de formule (II)

Composés de formule (II) dans laquelle :
Y représente un atome de fluor (préparations 1.26 et 1.27),
R₁ et R₂ sont en position 2 et 5 respectivement du .phényle.

### Préparation 1.26

### 4-(5-Cyclohexyl-2-methoxy-phenyl)-5-fluoro-thiazol-2-ylamine

A une solution de 2,5 g du composé obtenu à la préparation 1.3 décrite ci-dessus dans 30 mL de DMF, on ajoute à 0°C, 3,4 g de Selectfluor^{©} et le milieu est agité pendant 2 h à TA. Le milieu est hydrolysé par de l'ammoniac 2M dans l'éthanol, concentré puis dilué dans l'eau. Le brut est filtré, le solide est repris dans le DCM, lavé à l'eau puis à la soude 1M et avec une solution saturée de NaCl. Après séchage de la phase organique sur MgSO₄ et évaporation, le brut est purifié par flash-chromatographie.
On obtient 600 mg du composé attendu sous la forme d'une poudre blanche.
PF =159°C

### Préparation 1.27

### 4-(5-Propyl-2-methoxy-phenyl)-5-fluoro-thiazol-2-ylamine

Le composé est préparé selon la préparation 1.26 à partir du composé de la préparation 1.4.
PF =107°C

Analyse élémentaire :
%C 59.06 (théorie 58.63)
%H 5.85 (théorie 5.68)
%N 10.22 (théorie 10.52)

### Exemple 1 : (composé n° 9) N-[4-(2-méthoxy-5-propyl-phényl)-thiazol-2-yl]-N'-[4-(3-morpholin-4-yl-propyl)-pipérazin-1-yl]-urée

Composé de formule générale (I) dans laquelle:
R₁= 2-OMe ; R₂ = 5-nPr ; R₃ = 3-(morpholin-4-ylpropyl) ; a = 2

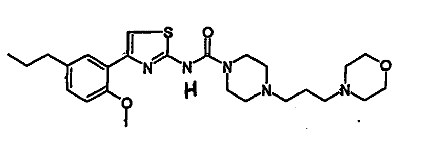

### 1.1. Préparation de la N-[4-(2-méthoxy-5-propyl-phényl)-thiazol-2-y]-N'-[4-(3-morpholin-4-yl-propyl)-pinpérazin-1-yl]-urée

A une solution de 0,1 g de 4-(2-Méthoxy-5-propyl-phenyl)-1,3-thiazol-2-amine, obtenue à la préparation 1.4 ci-dessus dans 2 mL de DMF, on additionne 0,18 g de DSC et on agite le milieu pendant 12 heures à TA. On ajoute 0,05 g de 1-(morpholin-4-ylpropyl)-pipérazine et le milieu est agité pendant 3 heures à TA. Le milieu est hydrolysé par une solution de NaHCO₃ saturée puis extrait dans le DCM. La phase organique est lavée à l'eau puis avec une solution saturée de NaCl, puis concentrée. Après séchage sur MgSO₄ , la solution est concentrée et purifiée par flash-chromatographie sur gel de silice. Le solide est repris dans le DCM, traité par une solution d'HCl 2M dans l'éther puis la suspension est évaporée pour donner 0,077 g du composé attendu sous la forme de son chlorhydrate.
PF = 229°C

### Exemple 2 : (composé n° 35) N-[4-(2-méthoxy-5-propyl-phényl)-thiazol-2-yl]-N'-[4-[2-(3-éthylamino-pyrrolidin-1-yl)-éthyl]-[1,4]diazepan-1-yl]-urée

Composé de formule générale (1) dans laquelle:
R₁ = 2-OCH₃ ; R₂ = 5-(CH₂)₂CH₃ ; R₃ = 4-[2-3-ethylamino-pyrrolidin-1-yl),ethyl] ; a=3

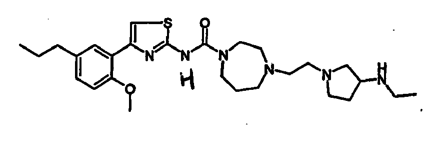

### 2.1 Préparation de la N-[4-(2-méthoxy-5-propyl-phényl-thiazol-2-yl]-N'-[4-(2-Chloro-acétyl)-[1,4]diazépan-1-yl]-urée

A une solution de 3,87 g de 4-(2-Méthoxy-5-propyl-phenyl)-1,3-thiazol-2-amine, obtenue à la préparation 1.4 ci-dessus dans 60 mL de DCM, on additionne 4g de DSC et on agite le milieu pendant 12 heures à TA. On ajoute 5,7 g de 2-Chloro-1-[1,4]diazepan-1-yl-ethanone et 3,26 mL de triéthylamine. Le milieu est agité pendant 3 heures à TA. Le milieu est hydrolysé par une solution de NaHCO₃ saturée puis extrait dans le DCM. La phase organique est lavée à l'eau puis à la saumure puis concentrée. Après séchage sur MgSO₄, la solution est concentrée pour donner 5,9 g du composé attendu. MH+ = 452 à t = 8,53 min

### 2.2. Préparation de la N-[4-(2-méthoxy-5 propyl-phényl)-thiazol-2-yl]-N'-[4-[2-(3-Acétylamino-pyrrolidin-1-yl-acétyl]-[1,4]diazépan-1-yl]-urée (composé n° 32)

Composé de formule générale (I) dans laquelle :
R₁ = 2-OCH₃ ; R₂ = 5-(CH₂)₂CH₃ ; R₃ = 4-[2-(3-Acetylamino-pyrrolidin-1-yl)-acetyl ;a=3

A une solution de 2 g de l'acide 4-(2-Chloro-acetyl)-[1,4]diazepane-1-carboxylique[4-(2-methoxy-5-propyl-phenyl)-thiazol-2-yl] -amide préparé à l'étape 2.1 dans 10 mL d'acétonitrile, on additionne 0,62 g de 3-acetamidopyrrolidine puis 0,612 g de K₂CO₃. Le milieu est agité à TA pendant 48 h. Après filtration, le milieu est lavé avec une solution de NaOH 1M puis à l'eau et avec une solution saturée de NaCl. Après séchage sur MgSO₄, la solution est concentrée puis purifiée par flash-chromatographie pour donner 0,95 g du produit attendu.
AE : %C = 56,94 %H = 6,89 %N =14,52(2 H₂O)
MH+ = 543 à t = 5,85 min.

### 2.3. Préparation de la N-[4-(2-méthoxy-5-propyl-phényl)-thiazol-2-yl]-N'-[4-2-(3-éthylamino-pyrrolidin-1-yl)-éthyl]-[1,4]diazépan-1-yl]-urée

A une solution de 0,79 g de l'acide 4-[2-(3-Acetylamino-pyrrolidin-1-yl)-acetyl]-[1,4]diazepane-1-carboxylique[4-(2-methoxy-5-propyl-phenyl)-thiazol-2-yl]-amide préparé à l'étape 2.2, dans 3 mL de DCM, on ajoute à 0°C 2,2 mL d'une solution à 65% de Red-al dans le toluène. Après 3 heures d'agitation à TA, le milieu est concentré puis repris au DCM et lavé à la soude 1M, à l'eau puis avec une solution saturée de NaCl. Après séchage sur MgSO₄, la phase organique est concentrée puis purifiée par flash-chromatographie pour donner 0,27 g du produit attendu.
MH+ = 515 à t = 8,77 min.

### Exemple 3 : (composé n° 76) (R)-N-[4-(5-cyclohexyl-2-méthoxy-phényl)-thiazol--2-yl]-N'-[4-{1-méthyl-pipéridin-3-ylméthyl)-pipérazin-1-yl]-urée

Composé de formule (I) dans laquelle :
R₁ = 2-OCH₃ ; R₂ = 5-CyHex R₃ = (R)-4-(1-Methy)-piperidin-3-ylmethyl); a=2

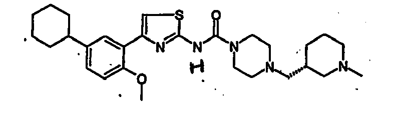

### 3.1. Préparation de l'acide (R)-3-Méthanesulfonyloxyméthyl-pipéridine-1-carboxylique tert-butyl ester

A une solution de 5 g d'acide (R) -3-Hydroxymethyl-piperidine-1-carboxylique tert-butyl ester dans 80 mL de DCM refroidie à 0°C, on ajoute 2,16 mL de chlorure de méthanesulfonyle puis 3,86 mL de triéthylamine. Le milieu est agité 1 h 30 à 0°C puis sont rajoutés 0,7 mL de triéthylamine et 0,54 mL de chlorure de méthanesulfonyle. Après 30 min à 0°C, le milieu est hydrolysé, la phase organique est lavée deux fois dans l'eau puis avec une solution saturée de NaCI puis séchée sur MgSO₄. Le milieu est évaporé pour donner 6,8 g d'une huile jaune pâle.

### 3.2. Préparation de l'acide (R)-3-(4-Benzyl-pipérazin-1-ylméthyl)-picéridine-1-carboxylique tert-butyl ester

Le brut obtenu à l'étape 3.1 est mis eh solution dans 75 mL de toluène. On ajoute 12:16.g de benzylpipérazine, le milieu réactionnel est scellé puis chauffé pendant 5 heures à 150°C. Après retour à TA, le milieu est dilué dans un mélange éther/pentane (1/1), lavé deux fois par une solution de NaHCO₃-saturée, deux fois dans l'eau puis avec une solution saturée de NaCl Après séchage sur MgSO₄ et évaporation, le brut est purifié par flash-chromatographie sur gel de silice pour donner 5,73 g du solide attendu.
MH+ = 374 à t = 5,26 min

### 3.3. Préparation du (R)-1-Benzyl-4-(1-méthyl-pipéridin-3-yl-méthyl)pipérazine

A une solution de 1 g de LiAIH₄ dans 45 mL de THF refroidie à 0°C, on ajoute 5 g du composé obtenu à l'étape 3.2 dissout dans 45 mL de THF. Le milieu est agité deux heures à TA puis refroidi à 0°C. On ajoute 0,96 mL d'eau puis 3 mL de NaOH 5M, le milieu est alors filtré et le solide est rincé à l'éther. Le filtrat est évaporé, repris dans l'éther, lavé deux fois par une solution de NaHCO₃ saturée puis avec une solution saturée de NaCl. Après séchage sur MgSO₄ et évaporation, on récupère 3,5 g du composé souhaité.
MH+= 288 à t = 5,68 min.

### 3.4. Préparation du (R)-1-(1-Méthyl-pinéridin-3-ylmethyl)-pipérazine

Une solution de 3,46 g du composé obtenu à l'étape 3.3 dans 100 mL de méthanol est hydrogénée en présence de 1,9 g de Pd/C à 10% humide sous 800 kPa de pression d'hydrogène à 40°C pendant 3 heures. Le milieu est filtré puis évaporé pour donner 2,26 g d'une huile incolore.

### 3.5. Préparation de la (R)-N-[4-(5-cyclohexyl-2-méthoxy-phényl)-thiazol-2-yl]-N'-[4-(1-méthyl-pipéridin-3-ylméthyl)-pipérazin-1-yl]-urée

La synthèse de ce composé est réalisée selon la procédure décrite dans l'exemple 1, à partir de la 4-(5-cyclohexyl-2-méthoxyphényl)-1,3-thiazol-2-amine décrite à la préparation 1.3 et du composé obtenu à l'étape 3.4.
PF =108°C ; [α]_{D}²⁵= -27° (c = 1,05 ; MeOH)

### Exemple 4 : (composé n° 70) N-[4-(5-cyclohexyl-2-méthoxy-phé nyl)-thiazol-2-yl]-N'-[4-(1-Isopropyl-pipéridin-3-yl)-pipérazin-1-yl]-urée

Composé de formule (I) dans laquelle :
R₁ = 2-OCH₃ ; R₂ = 5-CyHex ; R₃ = 4-(1-IsoPropyl-pipéridin-3-yl) ; a = 2

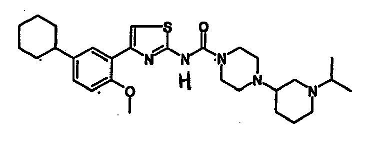

### 4.1. Préparation de l'acide 4-(1-Benzyl-pipéridin-3-yl)-pinérazine-1-carboxylique tert-butyl ester

A une suspension de 9,96 g de l'hydrate du monochlorhydrate de la 1-benzyl-3-piperidone en suspension dans 200 mL de DCM, on ajoute 20 mL d'une -solution de soude à 10%. Le milieu est agité, la phase organique est décantée puis lavée avec une solution saturée de NaCI. Après séchage sur MgSO₄, la phase organique est concentrée. La gomme obtenue est reprise dans 180 mL de DCE, on ajoute 10;1 g de Boc-pipérazine puis 15,9 g de NaBH(OAc)₃ et le milieu est agité 12 h à TA. Le milieu est concentré puis repris dans l'AcOEt. La phase organique est lavée deux fois avec une solution de NaHCO₃ saturée puis avec une solution saturée de NaCl. Après séchage sur MgSO₄, la phase organique est concentrée pour donner 18,63 g du produit attendu.
PF = 103°C

### 4.2. Préparation du 1-(1-Benzyl-pipéridin-3-yl)-pipérazine

A une solution de 9,2 g du composé obtenu à l'étape 4.1 dans 85 mL de DCM, on ajoute 30 g de TFA. Le milieu est agité 5 h à TA puis concentré. Le brut obtenu est repris dans le DCM puis lavé 4 fois avec une solution de soude 2M. La phase organique est lavée avec une solution saturée de NaCl. Après séchage sur MgSO₄, la phase organique est concentrée pour donner 6,32 g du produit attendu.
RMN ¹H: δ (ppm) = 7.28 (sél, 5H), 3.43 (sél, 2H), 2.88 (d, 1H), 2.70 (d, 1 H), 2.64 (m, 4H), 2.43-2.22 (m, 5H), 1.85-1.58 (m , 4H), 1.39 (ddd, 1 H), 1.15 (ddd, 1H).

### 4.3. Préparation de la N-[4-(5-cyclohexyl-2-méthoxy-phényl)-thiazol-2-yl]-N'-[4-(1-benzyl-pipéridin-3-yl)-pipérazin-1-yl]-urée (composé n° 68)

Composé de formule générale (I) dans laquelle:
R₁ = 2-OCH₃ ; R₂ = 5-CyHex ; R₃ = 4-(1-Benzyl-piperidin-3-yl) ; a = 2

La procédure est identique à celle décrite dans l'exemple 1 à partir de la 4-(5-cyclohéxyl-2-methoxyphényl)-1,3-thiazol-2-amine décrite à la préparation 1.3 et du composé obtenu à l'étape 4.2.
PF = 90°C

### 4.4. Préparation de la N-[4-(5-cyclohexyl-2-méthoxy-phényl-thiazol-2-yl]-N'-[4-Pipéridin-3-yl-pipérazin-1-yl]-urée (composé n° 69)

Composé de formule (I) dans laquelle :
R₁ = 2-OCH₃ ; R₂ = 5-CyHex ; R₃ = 4-(Piperidin-3-yl) ; a = 2

A une solution de 1,69 g du composé obtenu à l'étape 4.3 dans 10 mL de DCE, on ajoute à 0°C, 1,26 g de chloroethylchloroformate. Le milieu est ramené à TA puis chauffé à reflux pendant 45 min. Le milieu est évaporé puis repris dans 10. mL de MeOH et chauffé 1 h à reflux. Le brut est filtré, le solide est rincé à l'éther puis séché pour donner 1,27 g du composé attendu sous forme d'un - trichlorhydrate.
PF =-240°C
MH+ = 484 à 6,81 min

### 4.5. Préparation de la N-[4-(5-cyclohexyl-2-méthoxy-phényl)-thiazol-2-yl]-N'-[4-(1-Isopropyl-pipéridin-3-yl)-pipérazin-1-yl]-urée (composé n° 70)

Composé de formule (I) dans laquelle :
R₁ = 2-OCH₃ ; R₂ = 5-CyHex ; R₃ = 4=(1-Isopropyl-piperidin-3-yl) ; a = 2

A une solution de 0,2 g du composé obtenu à l'étape 4.4 dans 1,2 mL de DCE, on ajoute 0,05 mL d'acétone puis 0,15 g de NaBH(OAc)₃ et le milieu est agité 3 h à TA. On ajoute 0,1 mL de Et₃N puis 0,1 mg de NaBH(OAc)₃. Le milieu est agité 12 h à TA. Le milieu est concentré puis repris dans l'AcOEt. La phase organique est lavée deux fois avec une solution de NaHCO₃ saturée puis avec une solution saturée de NaCl. Après séchage sur MgSO₄, la phase organique est concentrée puis purifiée par flash-chromatographie pour donner 0,11 g du produit attendu.
PF = 130°C
MH+ = 526 à t = 7,06 min

### Exemple 5 : (composé n° 74) N-[4-(5-cyclohexyl-2-méthoxy-phényl)-thiazol-2-yl]-N'-[4-(1-Méthyl-pipéridine-2-carbonyl)-pipérazin-1-yl]-urée

Composé de formule (I) dans laquelle :
R_{1 =} 2-OCH₃; R₂ = 5-CyHex; R₃ = 4-(1-Methyl-piperidine,2-carbonyl) ; a=2

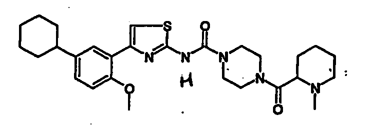

### 5.1. Préparation de l'acide 4-(1-Benzyloxycarbonyl-pipéridine-2-carbonyl): pipérazine-1-carboxylique tert-buyl ester

A une solution de 8 g de 1-Boc-piperazine dans 80 mL d'acétonitrile, on ajoute, à 0°C, 26,6 g de BOP, 13,6 g d'acide 1-(carbobenzyloxy)-2-pipéridine-carboxylique, puis 11,9 mL de triéthylamine. Le milieu est agité à TA pendant 12 heures puis concentré. Le milieu est repris dans l'AcOEt, lavé trois fois par une solution de Na₂CO₃ saturée puis avec une solution raturée de NaCl. Après séchage sur MgSO₄ et évaporation, on récupère 35,45 g de brut réactionnel. Il est repris dans le DCM puis lavé deux fois avec de la soude 5M, avec une solution saturée de NaCl, puis séché sur MgSO₄. Après évaporation, le solide est trituré dans le TBME, filtré, rincé au TBME puis séché pour donner 17,93 g du composé souhaité.
PF =102°C

### 5.2. Préparation de l'acide 4-(Pipéridine-2-carbonyl)-pipérazine-1-carboxylique tert-butyl ester

A une solution de 1,79 g du composé obtenu à l'étape 5.1 dans 14 mL d'EtOH, on ajoute sous atmosphère inerte, 3,9 mL de cyclohexadiene puis 1,3 g de Pd/C 10% humidifié à 50%. Le milieu est agité à TA pendant 24 heures puis filtré. Le filtrat est évaporé pour donner 1,02 g du composé souhaité.

### 5.3. Préparation dé l'acide 4-(1-Methyl-pipéridine-2-carbonyl)-pipérazine-1-carboxylique tert-butyl ester

A une solution de 0,99 g du composé obtenu à l'étape 5.2 dans 11 mL de DCE, on ajoute 0,54 mL de formaldéhyde aqueux à 37% puis 1,41 g de NaBH(OAc)₃ et la milieu est agité 12 h à TA. Le milieu est dilué dans le DCM, filtré sur coton. La phase organique est lavée deux fois avec une solution de NaHCO₃ saturée puis avec une solution saturée de NaCl. Après séchage sur MgSO₄, évaporation des solvants, on récupère 0,84 g du produit attendu.

### 5.4. Préparation du (1-Méthyl-pipéridin-2-yl)-pipérazine-1-yl-méthanone

A une solution de 0,84 g du composé obtenu à l'étape 5.3 dans 2 mL de DCM, on ajoute 2 mL de TFA. Le milieu est agité 6 h à TA. Le milieu est évaporé, repris plusieurs fois dans le DCM et évaporé pour entraîner le TFA. Le milieu est repris dans le DCM puis traité par une solution de NH₄OH à 10%. La phase organique est séchée sur MgSO₄ puis évaporée pour donner 0,15 g du produit attendu.

### 5.5. Préparation de la N-[4-(5-cyclohexyl-2-méthoxy-phényl)-thiazol-2-yl]-N'-[4-(1-Methyl-pipéridine-2-carbonyl)-pipérazin-1-yl]-urée

La procédure est identique à celle décrite dans l'exemple 1 à partir de la 4-(5-cyclohexyl-2-méthoxyphényl)-1,3-thiazol-2-amine décrite à la préparation 1.3 et du produit obtenu à l'étape 5.4.
PF =135°C

### Exemple 6 : (composé n° 88) N-[4-(5-cyctohexyl-2-méthoxy-phényl)-thiazol-2-yl]-N'-[4-(1-Méthyl-pipéridin-2-ylméthyl)-pipérazin-1-yl]-urée

Composé de formulé (I) dans laquelle :
R₁ = 2-OCH₃; R₂ = 5-CyHex; R₃ = 4-(1-Methyl-piperidin-2-ylmethyl); a = 2

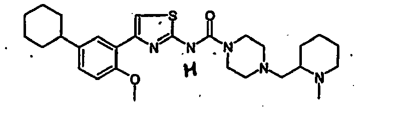

### 6.1. Préparation de l'acide 4-(1-Benzyloxycarbonyl-pipéridin-2-ylméthyl)-pipérazine-1-carboxylique tert-butyl ester

A une solution de 14 g du composé préparé à l'étape 5.1 dans 50 mL de THF, on ajoute, à 0°C, 162 mL d'une solution molaire de borane dans le THF sur une période de 30 min. Le milieu est agité à TA pendant 24 heures puis hydrolysé par addition d'eau à 0°C. Après dilution dans l'AcOEt, le milieu est lavé trois fois par une solution de Na₂CO₃ saturée puis avec une solution saturée de NaCI. Après séchage sur MgSO₄ et évaporation, on récupère 12,6 g du composé attendu.

### 6.2. Préparation de l'acide 4-Pipéridin-2-ylméthyl-pipérazine-1-carboxylique tert-butyl ester

A une solution de 6,43 g du composé obtenu à l'étape 6.1 dans 50 mL d'EtOH, on ajoute sous atmosphère inerte, 14,5 mL de cyclohexadiene puis 3,3 g de Pd/C 10% humidifié à 50%. Le milieu est agité à TA pendant 48 heures puis filtré: Le filtrat est évaporé pour donner 3,63 g du composé souhaité.

### 6.3. Préparation de l'acide 4-(1-Méthy(-pipéridin-2-ylméthyl)-pipérazine-1-carboxlique tert-butyl ester

A une solution de 3,34 g du composé préparé à l'étape 6.2 dans 40 mL de DCE, on ajoute 1,91 mL de formaldéhyde aqueux à 37% et quelques billes de tamis moléculaire 4Å puis 5 g de NaBH(OAc)₃ et le milieu est agité 48 h à TA. Le milieu est dilué dans le DCM, filtré sur coton. La phase organique est lavée deux fois avec une solution de NaHCO₃ saturée puis avec une solution saturée de NaCl. Après séchage sur MgSO₄, évaporation des solvants, on récupère 3,28 g du produit attendu.

### 6.4. Préparation du 1-(1-Méthyl-pipéridin-2-ylméthyl)-2-pipérazine

A une solution .de 3,28 g du composé obtenu à l'étape 6.3 dans 1 mL de dioxane, on ajoute 1 mL d'une solution 4 M d'HCl dans le dioxane. Le milieu est agité 48 h à TA. Le milieu est filtré, le solide est rincé à J'éther, puis séché pour donner 2,9 g du composé souhaité.

### 6.5. Préparation de la N-[4-(5-cyclohexyl-2-méthoxy-phényl)-thiazol-2-yl]-N'-[4-(1-Méthyl-pipéridin-2-ylméthyl)-pipérazin-1-yl]-urée

La procédure est identique à celle décrite dans l'exemple 1 à partir de la 4-(5-cyclohexyl-2-méthbxyphényl)-1,3-thiazol-2-amine décrite à la préparation 1.3 et du composé obtenu à l'étape 6.4.
PF = 103°C

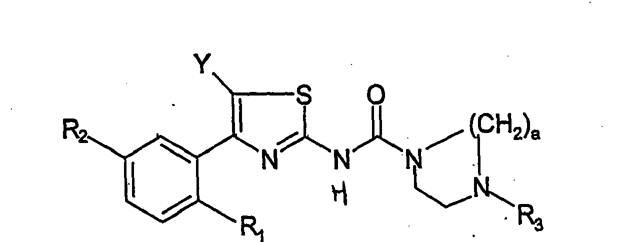

**Tableau II**

| N° | R₁ | R₂ | R₃ | Y | a | sel | PF (°C) M | Autre Analyse |
|---|---|---|---|---|---|---|---|---|
| 1 | -OCH₃ | -O(CH₂)₂CH₃ | 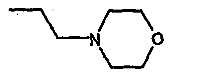 | H | 2 | - | 144°C | |
| 2 | - -OCH₃ | -O(CH₂)₂CH₃ | 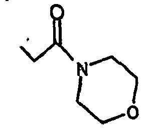 | H | 2 | - | 96°C | |
| 3 | -OCH₃ | -(CH₂)₂CH₃ | 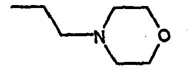 | H | 2 | - | 66°C | |
| 4 | -OCH₃ | -(CH₂)₂CH₃ | 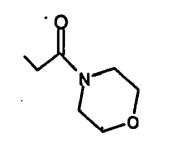 | H | 2 | - | 90°C | |
| 5 | -OCH₃ | 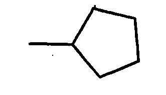 | 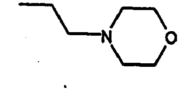 | H | 2 | - | 66°C | |
| 6 | -OCH₃ | -(CH₂)₃CH₃ | 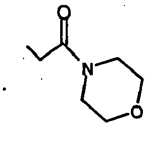 | H | 2 | - | 133°C | |
| 7 | -OCH₃ | -(CH₂)₃CH₃ | 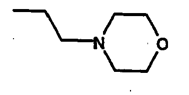 | H | 2 | - | 45°C | |
| 9 | -OCH₃ | -(CH₂)₂CH₃ | 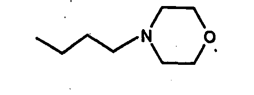 | H | 2 | HCl | 229°C | |
| 10 | -OCH₃ | -(CH₂)₂CH₃ | 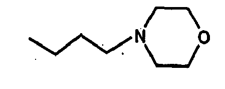 | H | 3 | HCl | 234°C | |
| 11 | -OCH₃ | -(CH₂)₂CH₃ | 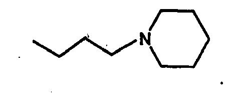 | H | 2 | HCl | 202°C | |
| 12 | -OCH₃ | -(CH₂)₂CH₃ | 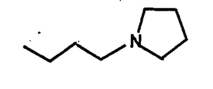 | H | 2 | HCl | 199°C | |
| 13 | -OCH₃ | -(CH₂)₂CH₃ | 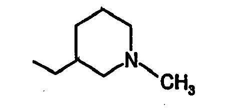 | H | 2 | HCl | 230°C | |
| 14 | -OCH₃ | -(CH₂)₂CH₃ | 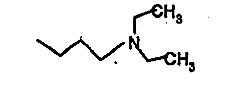 | H | 2 | HCl | 145°C | |
| 15 | -OCH₃ | -(CH₂)₂CH₃ | 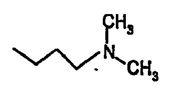 | H | 2 | HCl | 245°C | |
| 16 | -OCH₃ | -(CH₂)₂CH₃ | 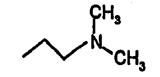 | H | 2 | HCl | 239°C | |
| 17 | -OCH₃ | -(CH₂)₂CH₃ | 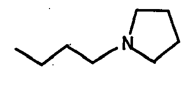 | H | 3 | HCl | 223°C | |
| 18 | -OCH₃ | -(CH₂)₂CH₃ | 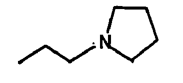 | H | 2 | HCl | 245°C | |
| 19 | -OCH₃ | -(CH₂)₂CH₃ | 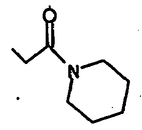 | H | 2 | HCl | 180°C | |
| 20 | -OCH₃ | -(CH₂)₂CH₃ | 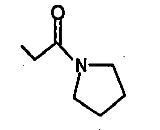 | H | 2 | HCl | 181°C | |
| 21 | -OCH₃ | -(CH₂)₂CH₃ | 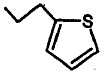 | H | 2 | HCl | 209°C | |
| 22 | -OCH₃ | -(CH₂)₂CH₃ | 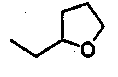 | H | 2 | HCl | 200°C | |
| 23 | -OCH₃ | -(CH₂)₂CH₃ | 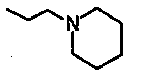 | H | 2 | HCl | 242°C | |
| 24 | -OCH₃ | -(CH₂)₂CH₃ | 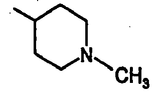 | H | 2 | HCl | 248°C | |
| 25 | -OCH₃ | 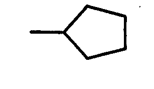 | 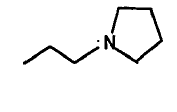 | H | 2 | - | 114°C | |
| 26 | -OCH₃ | -(CH₂)₃CH₃ | 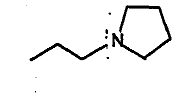 | H | 2 | - | 65°C | |
| 27 | -OCH₃ | -O(CH₂)₂CH₃ | 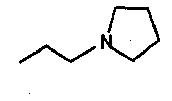 | H | 2 | - | 70°C | |
| 28 | -OCH₃ | -(CH₂)₂CH₃ | 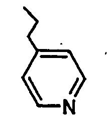 | H | 2 | - | 82°C | |
| 29 | -OCH₃ | 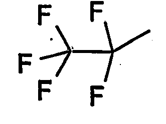 | 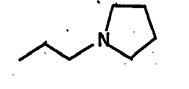 | H | 2 | HCl | MH+ = 534 à 6,14 min. | |
| 30 | -OCH₃ | 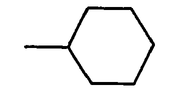 | 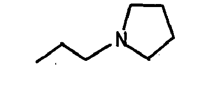 | H | 2 | - | MH+= 498 à 6,52 min | |
| 31 | -OCH₃ | -(CH₂)₂CH₃ | 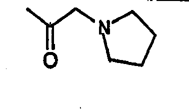 | H | 2 | - | 89°C | |
| 32 | -OCH₃ | -(CH₂)₂CH₃ | 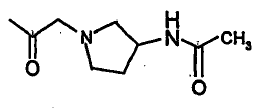 | H | 3 | - | MH+= 543 à 5,85 min | |
| 33 | -OCH₃ | -(CH₂)₂CH₃ | 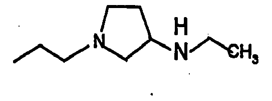 | H | 2 | - | MH+= 501 à 5,03 min | |
| 34 | -OCH₃ | -(CH₂)₂CH₃ | 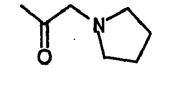 | H | 3 | - | MH+ = 486 à 6,04 min | |
| 35 | -OCH₃ | -(CH₂)₂CH₃ | 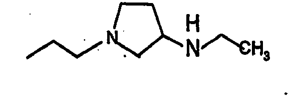 | H | 3 | HCl | MH+ = 515 à 8,77 min | |
| 36 | -OCH3 | -(CH₂)₂CH₃ | 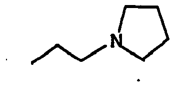 | F | 2 | - | 63°C | |
| 37 | -OCH3 | -(CH₂)₂CH₃ | 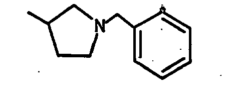 | H | 2 | - | 63°C | |
| 38 | -OCH₃ | -(CH₂)₅CH₃ | 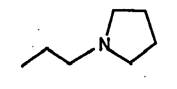 | H | 2 | HCl | MH+=500 à 6,99min | |
| 39 | -OCH₂CH₃ | -CH₂CH₃ | 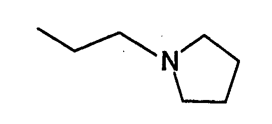 | H | 2 | - | 96°C | |
| 40 | -OCH₂CH₃ | -(CH₂)₃CH₃ | 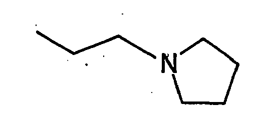 | H | 2 | - | 130°C | |
| 41 | -OCH₂CH₃ | 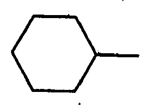 | 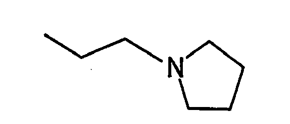 | H | 2 | - | 89°C | |
| 42 | -OCH₃ | -CH₂CH₃ | 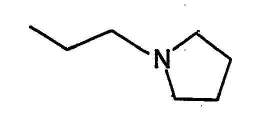 | H | 2 | - | 100°C | |
| 43 | -OCH₃ | 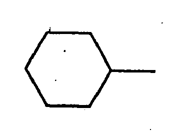 | 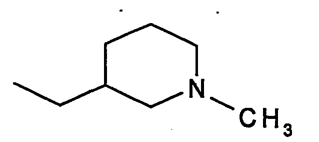 | H | 2 | - | 106°C | |
| 44 | -OCH₃ | 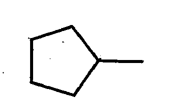 | 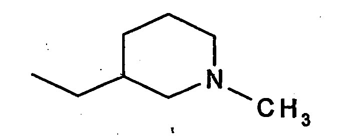 | H | 2 | - | 98°C | |
| 45 | -OCH₃ | -(CH₂)₃CH₃ | 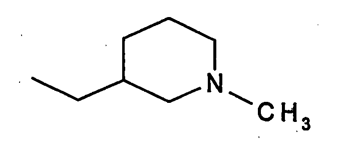 | H | 2 | - | 90°C | |
| 46 | -OCH₃ | -(CH₂)₃CH₃ | 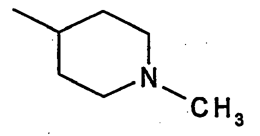 | H | 2 | - | 69°C | |
| 47 | -OCH₃ | 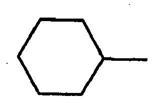 | 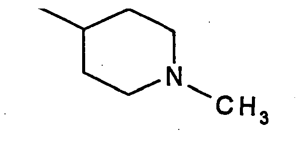 | H | 2 | - | 103°C | |
| 48 | -OCH₃ | 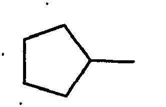 | 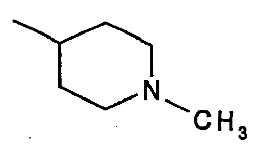 | H | 2 | - | 98°C | |
| 49 | -OCH₂CH₃ | 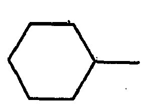 | 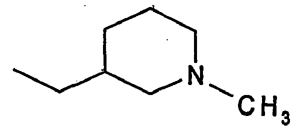 | H | 2 | - | 109°C | |
| 50 | -OCH₂CH₃ | 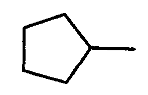 | 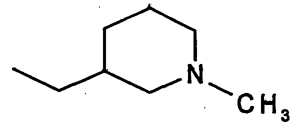 | H | 2 | - | 108°C | |
| 51 | -OCH₂CH₃ | 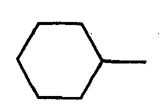 | 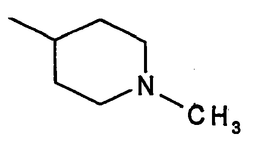 | H | 2 | - | 106°C | |
| 52 | -OCH₂CH₃ | 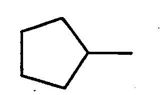 | 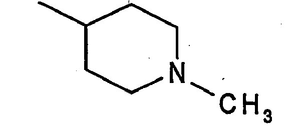 | H | 2 | - | 96°C | |
| 53 | -OCH₂CH₃ | 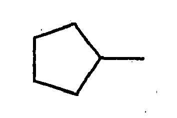 | 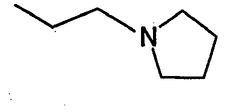 | H | 2 | - | 75°C | |
| 54 | -OCH₂CH₃ | -(CH₂)₃CH₃ | 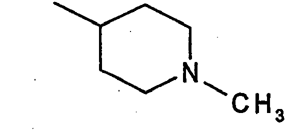 | H | 2 | - | 140°C | |
| 55 | -OCH₃ | 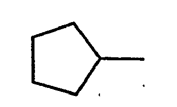 | 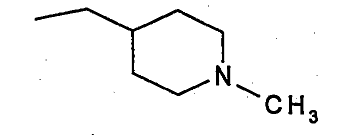 | H | 2 | - | 102°C | |
| 56 | -OCH₂CH₃ | -(CH₂)₃CH₃ | 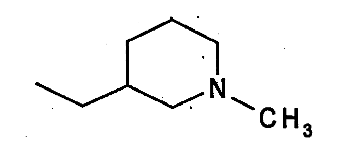 | H. | 2 | - | 91°C | |
| 57 | -OCH₂CH₃ | 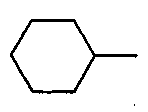 | 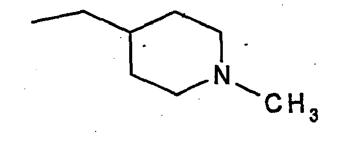 | H | 2 | - | 110°C | |
| 58 | -OCH₃ | 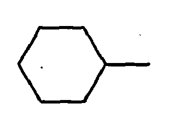 | 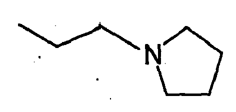 | F | 2 | HCl | MH+=516 à 6,79 min | |
| 59 | -OCH₃ | 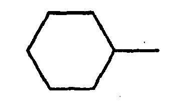 | 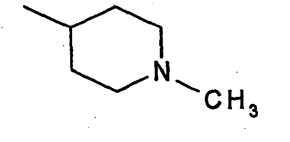 | F | 2 | HCl | MH+= 515 à 6,01 min | |
| 60 | -OCH₃ | 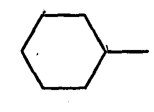 | 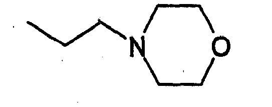 | F | 2 | HCl | MH+= 532 à 6,91 min | |
| 61 | -OCH₃ | 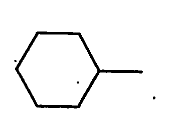 | 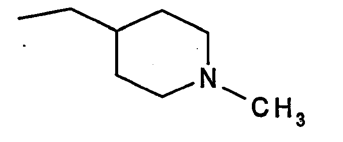 | H | 2 | - | 118°C | |
| 62 | -OCH₃ | -(CH₂)₃CH₃ | 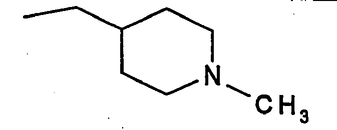 | H | 2 | - | 84°C | |
| 63 | -OCH₃ | 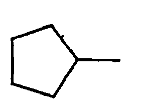 | 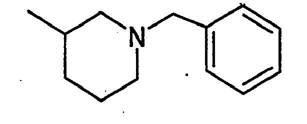 | H | 2 | - | 79°C | |
| 64 | -OCH₃ | 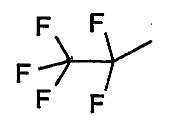 | 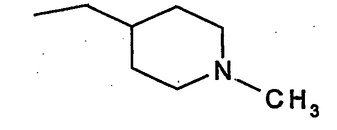 | H | 2 | - | 97°C | |
| 65 | -OCH₃ | 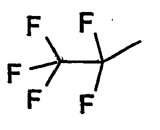 | 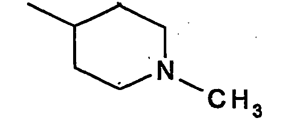 | H | 2 | - | 172°C | |
| 66 | -OCH₃ | 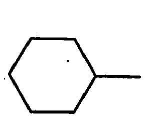 | 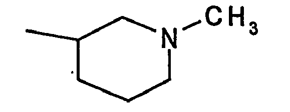 | H | 2 | - | 107°C | |
| 67 | -OCH₃ | 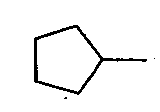 | 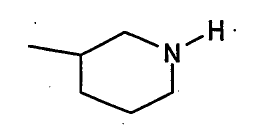 | H | 2 | - | 83°C | |
| 68 | -OCH₃ | 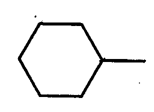 | 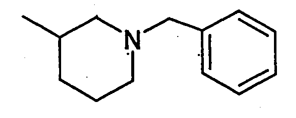 | H | 2 | - | 90°C | |
| 69 | -OCH₃ | 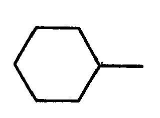 | 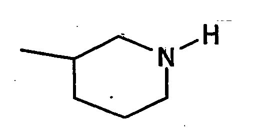 | H | 2 | 3 HCl | 240°C | |
| 70 | -OCH₃ | 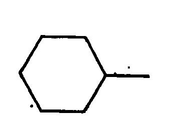 | 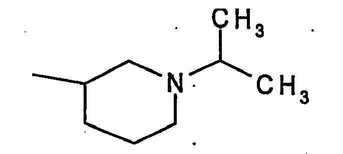 | H | 2 | - | 130°C | |
| 71 | -OCH₃ | 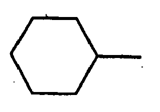 | 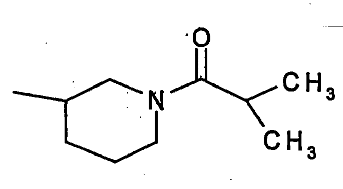 | H | 2 | - | 127°C | |
| 72 | -OCH₃ | 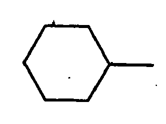 | 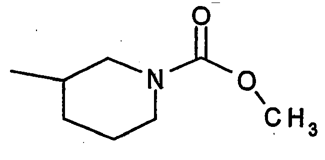 | H | 2 | - | 125°C | |
| 73 | -OCH₃ | 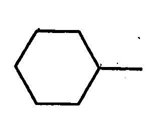 | 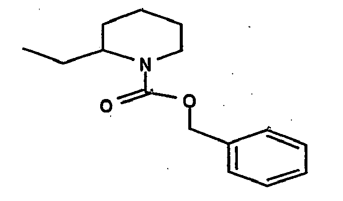 | H | 2 | - | 122°C | |
| 74 | -OCH₃ | 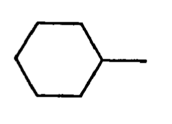 | 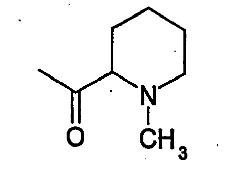 | H | 2 | - | 135°C | |
| 75 | -OCH₃ | 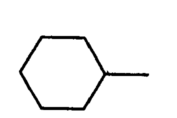 | 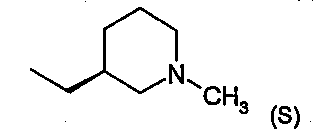 | H | 2 | - | 107°C | |
| 76. | -OCH₃ | 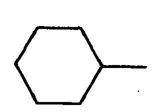 | 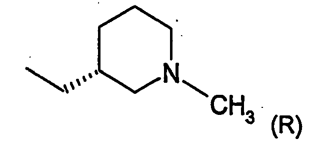 | H | 2 | - | 108°C | |
| 77 | -OCH₃ | -(CH₂)₃CH₃ | 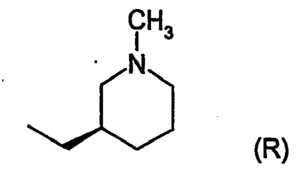 | H | 2 | - | 85°C | |
| 78 | -OCH₃ | 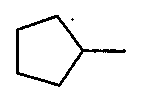 | 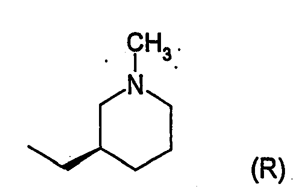 | H | 2 | - | 94°C | |
| 79 | -OCH₃ | -(CH₂)₅CH₃ | 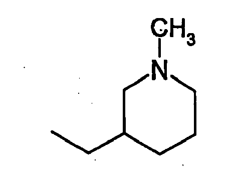 | H | 2 | - | 68°C | |
| 80 | -OCH₂CH₃ | -(CH₂)₅CH₃ | 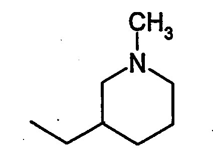 | H | 2 | - | 65°C | |
| 81 | -OCH₂CH₃ | -(CH₂)₃CH₃ | 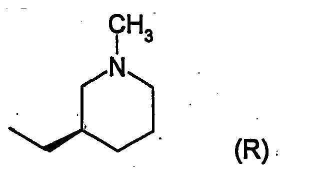 | H | 2 | - | 75°C | |
| 82 | -OCH₃ | 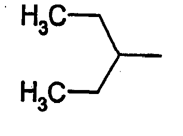 | 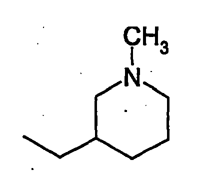 | H | 2 | - | 85°C | |
| 83 | -OCH₂CH₃ | -(CH₂)₂CH₃ | 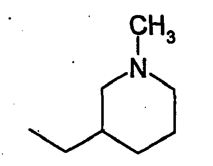 | H | 2 | - | 74°C | |
| 84 | -OCH₂CH₃ | 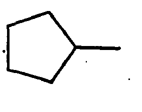 | 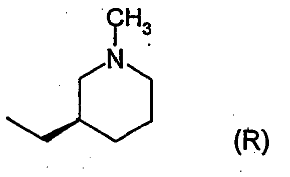 | H | 2 | - | 92°C | |
| 85 | -O(CH₂)₂CH₃ - | -CH₂CH₃ | 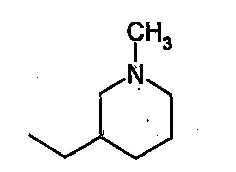 | H | 2 | - | 73°C | |
| 86 | -OCH₃ | -CH₂CH₃ | 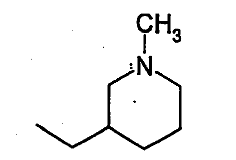 | H | 2 | - | 86°C | |
| 87 | -OCH₂CH₃ | 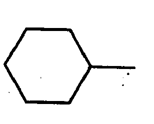 | 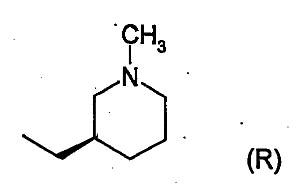 | H | 2 | - | 98°C, 103°C | |
| 88 | -OCH₃ | 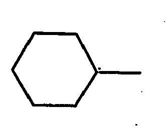 | 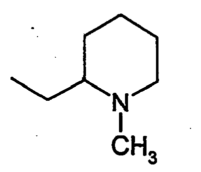 | H | 2 | - | 103°C | |
| 89 | -OCH₃ | 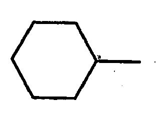 | 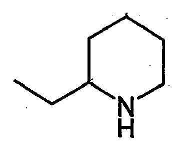 | H | 2 | - | 121°C | |
| 90 | -OCH₃ | 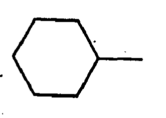 | 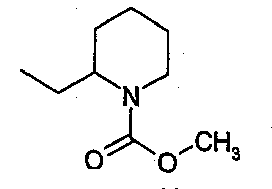 | H | 2 | - | 119°C | |
| 91 | -OCH₃ | -CH₃ | 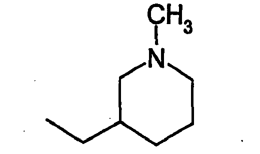 | H | 2 | - | 95°C | |
| 92 | -OCH₃ | 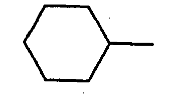 | 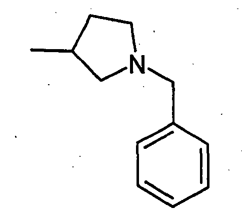 | H | 2 | - | 85°C | |
| 93 | -OCH₃ | 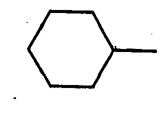 | 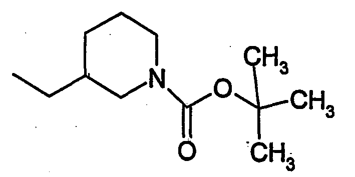 | H | 2 | - | 125°C | |
| 94 | -OCH₃ | 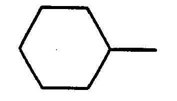 | 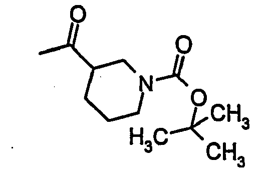 | H | 3 | - | 11.2°C | |
| 95 | -OCH₃ | 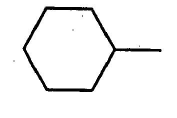 | 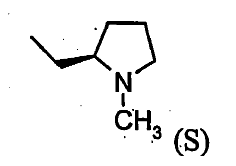 | H | 2 | - | 101°C | |
| 96 | -OCH₃ | 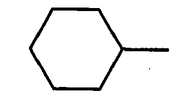 | 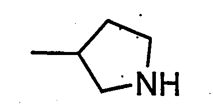 | H | 2 | - | 215°C | |
| 97 | -OCH₃ | 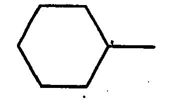 | 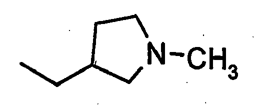 | H | 2 | - | 103°C | |
| 98 | -OCH₃ | 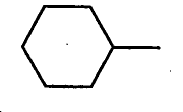 | 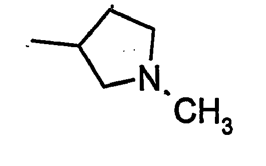 | H | 2 | - | 106°C | |
| 99 | -OCH₃ | 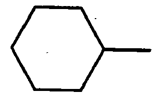 | 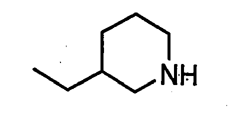 | H | 2 | - | 128°C | |
| 100 | -OCH₃ | 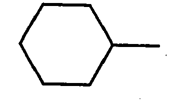 | 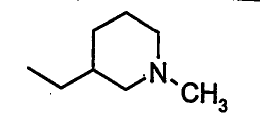 | H | 3 | - | 88°C | |
| 101 | -OCH₃ | 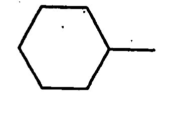 | 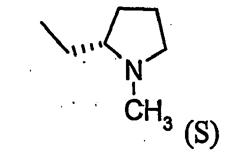 | H | 2 | - | 122°C | |
| 102 | -OCH₃ | 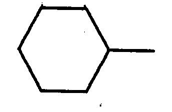 | 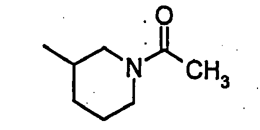 | H | 2 | - | 146°C | |
| 103 | -OCH₃ | 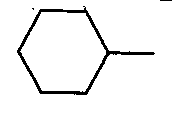 | 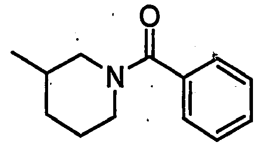 | H | 2 | - | 132°C | |
| 104 | -OCH₃ | 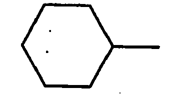 | 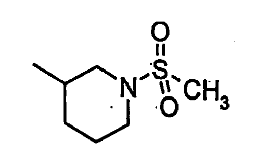 | H | 2 | - | 132°C | - |
| 105 | -OCH₃ | 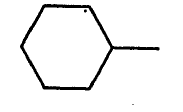 | 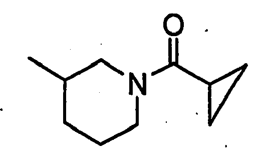 | H | 2 | - | 119°C | - |
| 106 | -OCH₃ | 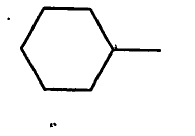 | 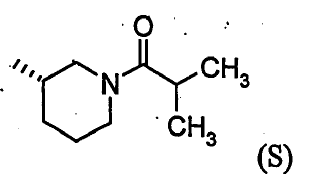 | H | 2 | - | 118°C | - |
| 107 | -OCH₃ | 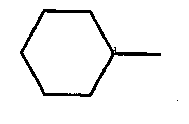 | 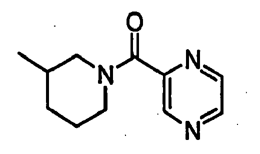 | H | 2 | - | - | MH+= 590 à 7;52 min |
| 108 | -OCH₃ | 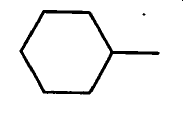 | 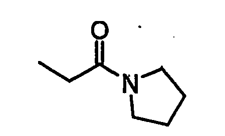 | H | 2 | - | - | C:63,17 H: 7,20 N : 13,51 |
| 109 | -OCH₂CH₃ | 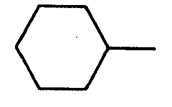 | 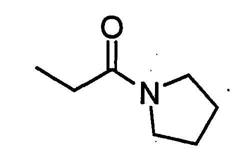 | H | 2 | - | 111°C | - |
| 110 | -OCH₂CH₃ | 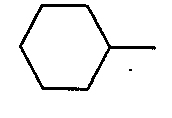 | 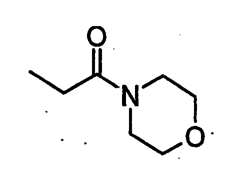 | H | 2 | - | 113°C | - |
| 111 | -OCH₃ | 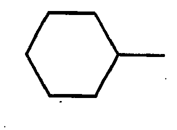 | 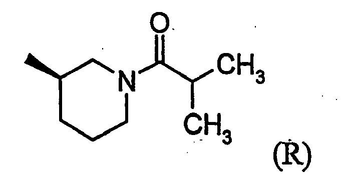 | H | 2 | - | 98°C | α_{D} = -6.2° c=1,0 M MeOH |
| 112 | -OCH₃ | 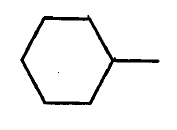 | 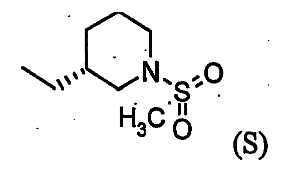 | H | 2 | - | 158°C | - |
| 113 | -OCH₃ | 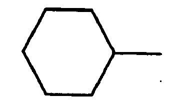 | 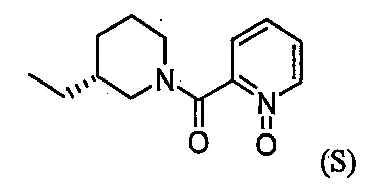 | H | 2 | - | 158°C | |
| 114 | -OCH₃ | 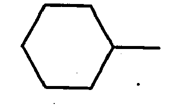 | 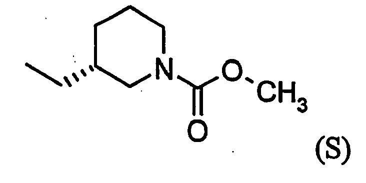 | H | 2 | - | 125°C | |
| 115 | -OCH₃ | 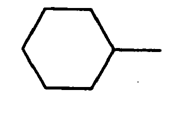 | 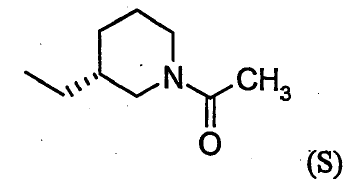 | H | 2 | - | 157°C | |
| 116 | -OCH₃ | 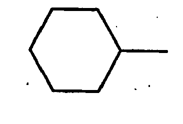 | 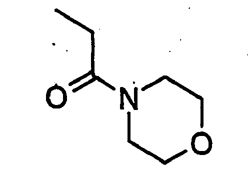 | H | 2 | - | 119°C | |
| 117 | -OCH₃ | 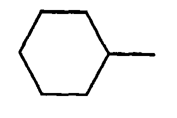 | 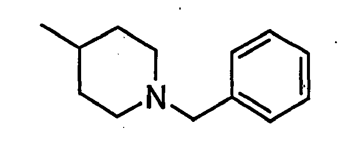 | H | 2 | - | 81°C | |
| 118 | -OCH₃ | 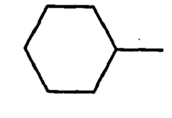 | 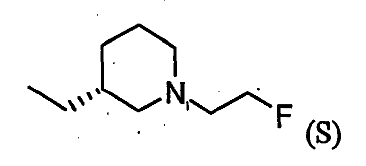 | H | 2 | - | 91°C | |
| 119 | -OCH₃ | 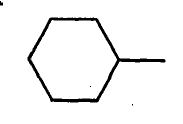 | 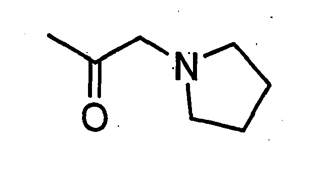 | H | 2 | - | 117°C | |
| 120 | -OCH₃ | 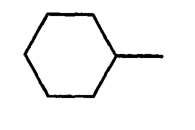 | 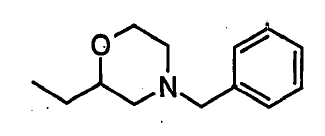 | H | 2 | - | 78°C | |

Les composés selon l'invention ont fait l'objet d'essais pharmacologiques permettant de déterminer leur effet modulateur de l'activité des récepteurs aux chimiokines. Les chimiokines sont des protéines de bas poids moléculaire qui appartiennent à la famille des cytokines proinflammatoires et sont impliquées dans le chimiotactisme des leucocytes et des cellules, endothéliales. Les chimiokines contrôlent de nombreux processus biologiques et sont associées à des désordres inflammatoires apparaissant lors des états de stress, lors de blessures ou d'infections ; la modulation des effets des chimiokines permet de prévenir ou de traiter des pathologies comme l'asthme, l'arthrite, les allergies, les maladies auto-immunes, l'athérosclérose ou l'angiogénèse (C.D. Paavola et al., J. Biol. Chem., 1998, 273, (50), 33157-33165).

Parmi les chimiokines, on distingue le hMCP-1 (de l'anglais human Monocyte Chemotactic Protein) qui appartient au groupe des CC chimiokines et qui est un agoniste naturel du récepteur CCR2b.

On a mesuré l'activité inhibitrice des composés selon l'invention sur des cellules exprimant le récepteur CCR2b humain. La concentration d'agoniste naturel hMCP-1 qui inhibe 50 % (Cl₅₀) de l'activité du récepteur CCR2b est de 0,57 nM. Les composés selon l'invention présentent une CI₅₀ généralement inférieure à 0,1 µM.

Par exemple, le composé n° 14 a présenté une Cl₅₀ de 0,0033 µM;
le composé n° 28 a présenté une Cl₅₀ de 0,028 µM;
le composé n° 55 a présenté une Cl₅₀ de 0,014 µM.

On a également mesuré l'inhibition du chimiotactisme sur des cellules monocytaires THP-1 humaines (commercialisées par DSMZ - Allemagne) en utilisant une technique adaptée de celle décrite par A. Albini et al., Cancer Res., 1987, 47, 3239-3245. Dans ces conditions le hMCP-1 présente une Cl₅₀ de 6 nM. Les composés selon l'invention présentent une Cl₅₀ généralement inférieure à 1 µM.

L'inhibition du chimiotactisme par les composés selon l'invention est le signe de leur activité antagoniste sur les récepteurs des chimiokines et en particulier du CCR2b. Il apparaît donc que les composés selon l'invention sont des antagonistes de l'effet des chimiokines, en particulier du hMCP-1.

On a également mesuré l'activité inhibitrice des composés selon l'invention sur des PBMC (Peripheral Blood Mononuclear Cells) infectés par le virus VIH-1 Bal, selon une technique adaptée de celle décrite par V. Dolle et col., J. Med. Chem., 2000, 43, 3949, 3962. Selon.cette technique, les PBMC sont infectés par le VIH-1 Bal puis les composés à tester sont ajoutés dans le milieu de culture pendant 5 jours. Au terme de cette exposition, on mesure dans le surnageant le taux de transcriptase reverse qui est corrélé avec le niveau de réplication virale dans les cellules. Dans ces conditions, l'AZT, molécule de référence qui inhibe la réplication virale présente une Cl₅₀ inférieure à 1 µM, Des composés selon l'invention présentent également des Cl₅₀ inférieures à 1µM. Par exemple, le composé n° 30 a montré une Cl₅₀ de 0,6 µM.

Les composés selon l'invention peuvent donc être utilisés pour la préparation de médicaments, en particulier de médicaments antagonistes de l'effet des chimiokines.

Ainsi, selon un autre de ses aspects, la présente invention a pour objet des médicaments qui comprennent un composé de formule (I), ou un sel d'addition de ce dernier à un acide pharmaceutiquement acceptable, ou encore un hydrate ou un solvat.

Ces médicaments trouvent leur emploi en thérapeutique, notamment dans la prévention et le traitement de différentes pathologies telles que :
- les maladies et les syndromes immuno-inflammatoires aigus et chroniques comme l'athérosclérose, les resténoses, les maladies pulmonaires chroniques, en particulier COPD (de l'anglais chronic obstructive pulmonary disease); le syndrome de détresse respiratoire ; l'hyperactivité bronchique; les colites; la silicose ; les pathologies fibreuses, les fibroses pulmonaires, les fibroses kystiques ; les infections virales ou bactériennes, SIDA, méningite, malaria, lèpre, tuberculose, herpès, infections par cytomégalovirus; les chocs septiques, la septicémie, les chocs endotoxiques ; les rejets de greffes ; les pathologies osseuses tettes que l'ostéoporose, les ostéoarthrites ; les conjonctivites ; les dermatites atypiques ou de contact; les eczémas ; les glomérulonéphrites ; les pancréatites ; les colites ulcéreuses, les maladies auto-immunes comme la polyarthrite rhumatoïde, la sclérose en plaques, la sclérose amyotrophique latérale, la maladie de Crohn, le lupus érythémateux, la sclérodermie, le psoriasis; la maladie de Parkinson ; la maladie d'Alzheimer ; le diabète ; la cachexie; l'obésité;
- le traitement de la douleur, en particulier neuropathique et inflammatoire;
- les maladies allergiques comme les maladies respiratoires allergiques, l'asthme, les rhinites, l'hypersensibilité pulmonaire, l'hypersensibilité retardée;
- les maladies et les désordres dans lesquels les processus angiogéniques sont impliqués comme les cancers (angiogénèse intratumorale), les maladies rétiniennes (dégénérescence maculaire liée à l'age: DMLA);
- les pathologies cardiaques : choc hémodynamique ; les ischémies cardiaques ; les attaques de réinfusion post-ischémique; ; l'infarctus du myocarde, la thrombose coronarienne, l'insuffisance cardiaque, l'angine de poitrine.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (1) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et- les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :
- Composé selon l'invention: 50,0 mg
- Mannitol: 223,75 mg
- Croscaramellose sodique: 6,0 mg
- Amidon de maïs: 15,0 mg
- Hydroxypropyl-méthylcellulose: 2,25 mg
- Stéarate de magnésium: 3,0 mg
Par voie orale, la dose de principe actif administrée par jour peut atteindre 0,1 à 1000 mg/kg, en une ou plusieurs prises .

Il peut y avoir des cas particuliers où des dosages plus élevés ou plus faibles sont appropriés; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé de formule (1) suivante : 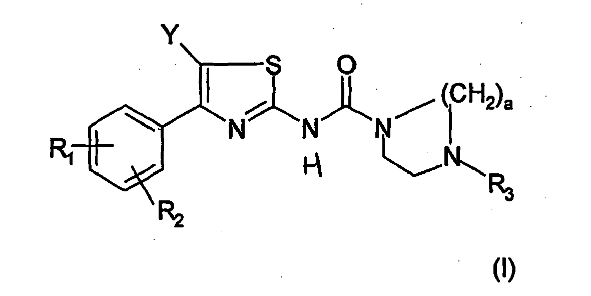 dans laquelle :
- (i) R₁ est sélectionné dans le groupe constitué par H, halogène; (C₁-C₈)alkyle, trifluoro(C₁-C₄)alkyle, -OH, -O-(C₁-C₈)alkyle, -O-trifluoro(C₁-C₈)alkyle, -O-(C₃-C₁₀)cycloalkyl(C₁-C₈)alkyle, -O-(C₃-C₁₀)cycloalkyle, -O-CH₂-CH=CH₂, et (C₁-C₄)alkylthio ;
- (ii) R₂ est sélectionné dans le groupe constitué par H, halogène, -OH, (C₁-C₈)alkyle, trifluoro(C₁-C₄)alkyle, perfluoro(C₁-C₄)alkyle, (C₃-C₁₀)cycloalkyle, -O-(C₁-C₈)alkyle, -O-(C₃-C₁₀)cycloalkyl(C₁-C₈)alkyle, -O-(C₃-C₁₀)cycloalkyle, -O-CH₂-CH=CH₂ , et (C₃-C₈)cycloalkyl(C₁-C₈)alkyle;
- (iii) Y représente un atome d'hydrogène ou un halogène ;
- (iv) R₃ représente :
a1) un groupe de formule -(CH₂)ₚ-A
dans laquelle p représente 0, 1, 2, 3 ou 4 et :
- lorsque p représente 2, 3 ou 4, A représente un groupe de formule : 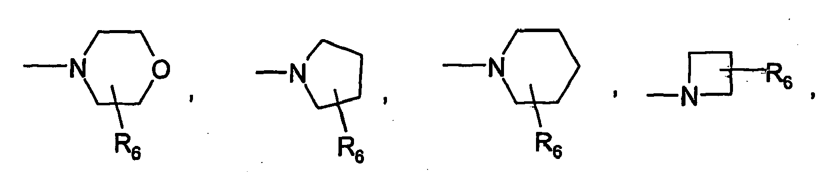 ou -NR₄R₅,
dans laquelle R₆ est sélectionné dans le groupe constitué par H, F, -(C₁-C₄)alkyle, -(CH₂)ₙOH, -(CH₂)ₙO(C₁-C₄)alkyle, et -(CH₂)ₙNR₄R₅, où n représente 0, 1 ou 2 et R₄ et R₅ représentent indépendamment l'un de l'autre un atome d'hydrogène, un groupe (C₁-C₈)alkyle, un groupe -CO(C₁-C₄)alkyle, ou -CO-O-(C₁-C₈)alkyle;
- ou, lorsque p représente 1, 2, 3 ou 4, A représente un groupe de formule: 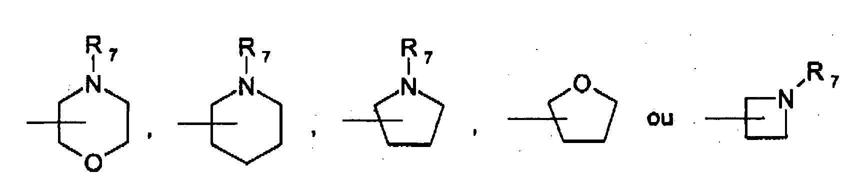 dans laquelle R₇ est sélectionné dans le groupe constitué par H, (C₁-C₈)alkyle, -CO-(C₁-C₈)alkyle, benzyle, -CO-O-(C₁-C₈)alkyle, -CO-O-benzyle, -CO-phenyl, -CO-hétéroaryle, -CO-(C₃-C₁₀)cycloalkyle, -SO₂-(C₁-C₈)alkyle, -SO₂-(C₃-C₆)cycloalkyle, et -SO₂-hétéroaryle;
- ou, lorsque p représente 0,1,2, 3 ou 4, A représente un groupe de formule: 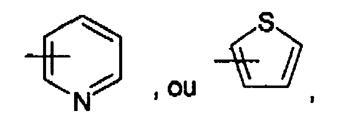 ledit groupe étant éventuellement substitué par un groupe (C₁-C₄)alkyle;
a2) un groupe de formule -(CH₂)p-CO-A
dans laquelle p représente 1, 2, 3 ou 4,
- A représente un groupe de formule : 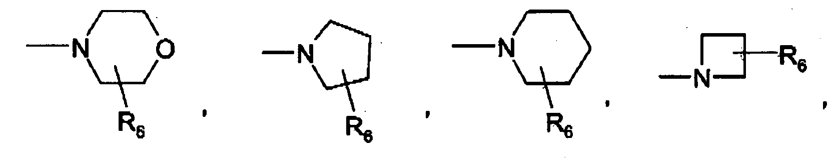 ou -NR₄R₅,
dans laquelle R₄, R₅ et R₆ sont tels que définis dans ce qui précède;
a3) un groupe de formule -CO(CH₂)p-A
dans laquelle p représente 0, 1, 2, 3 ou 4,
- lorsque p représente 1, 2, 3 ou 4, A représente un groupe de formule : 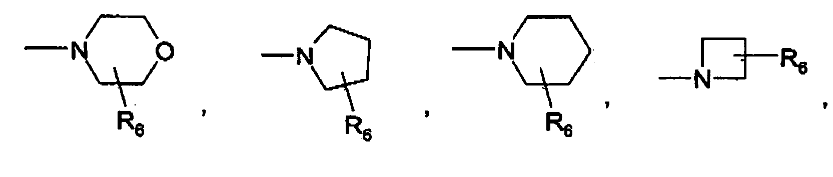 ou -NR₄R₅,
dans laquelle R₄, R₅ et R₆ sont tels que définis dans ce qui précède;
- ou, lorsque p représente 0,1,2, 3 ou 4, A représente un groupe de formule : 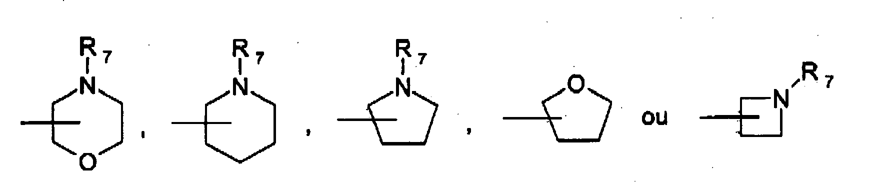 dans laquelle R₇ est tel que défini précédemment ;
- ou, lorsque p représente 0,1, 2, 3 ou 4, A représente un groupe de formule: 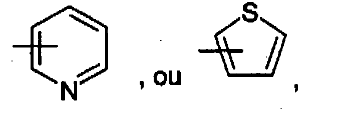 ledit groupe étant éventuellement substitué par un groupe (C₁-C₄)alkyle;
a4) un groupe -B
dans lequel B représente un groupe de formule : 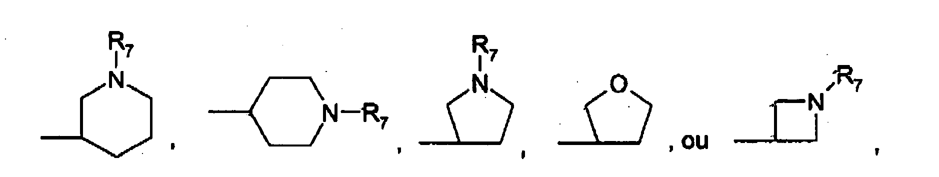 dans laquelle R₇ est tel que défini précédemment:
- (v) a représente 2 ou 3;
à l'état de base ou de sel d'addition à un acide, ainsi qu'à l'état d'hydrates ou de solvates.

2. Composé selon la revendication 1, de formule (I.a) suivante : 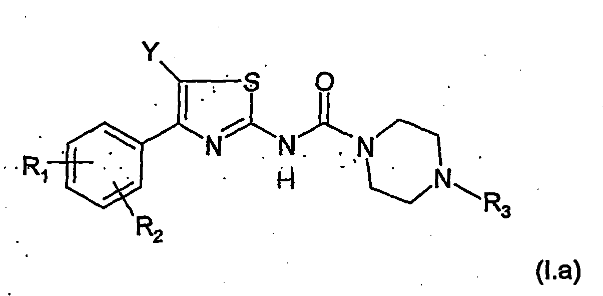 dans laquelle:
R₁, R₂, R₃ et Y sont tels que définis précédemment,
à l'état de base ou de sel d'addition à un acide, ainsi qu'à l'état d'hydrates ou de solvats.

3. Composé selon la revendication 1, de formule (I.b) suivante : 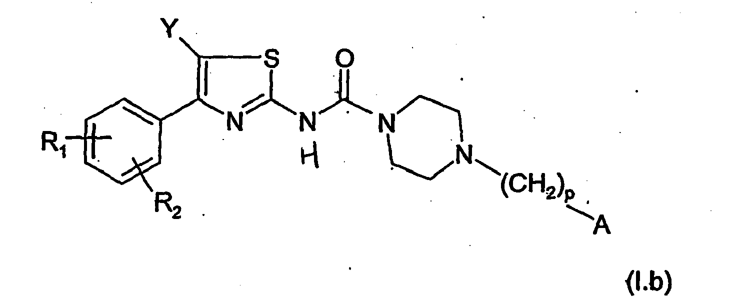 dans laquelle R₁, R₂ , Y, p et A sont tels que définis précédemment,
à l'état de base ou de sel d'addition à un acide, ainsi qu'à l'état d'hydrates, ou de solvats.

4. Composé selon la revendication 1, de formule (I.c) suivante : 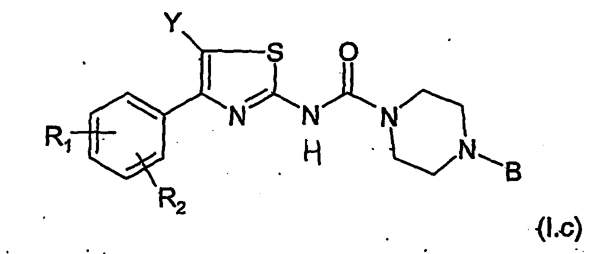 dans laquelle R₁, R₂, Y et B sont tels que définis précédemment,
à l'état de base ou de sel d'addition à un acide, ainsi qu'à l'état d'hydrates ou de solvats.

5. Composés selon l'une quelconque des revendications 1 ou 2, choisis parmi :
• (R)-N-[4-(5-cyclohexyl-2-méthoxy-phényl)-thiazol-2-yl]-N'-[4-(1-méthyl-pipéridin-3-ylméthyl)-pipérazin-1 -yl]-urée,
• N-[4-(2-méthoxy-5-propoxy-phényl)-thiazol-2-yl]-N'-[4-(2-morpholin-4-yl-éthyl)-pipérazin-1-yl]-urée,
• N-[4-(2-méthoxy-5-propyl-phényl)-thiazol-2-yl]-N' -[4-(3-pipéridin-1-yl-propyl)-pipérazin-1-yl]-urée,
• N-[4-(2-méthoxy-5-propyl-phényl)-thiazol-2-yl]-N'-[4-(2-diméthylamino-éthyl)-pipérazin-1-yl]-urée,
• N-[4-(2-méthoxy-5-propyl-phényl)-thiazol-2-yl]-N'-[4-(2-oxo-2-pyrrolidin-1-yl-éthyl)-pipérazin-1-yl]-urée,
• N-[4-(2-méthoxy-5-propy)-phényl)-thiazol-2-yl]-N'-[4-(2-thiophén-2-yl-éthyl)-pipérazin-1-yl]-urée,
• N-[4-(2-méthoxy-5-propyl-phényl)-thiazol-2-yl]-N'-[4-[2-(tétrahydro-furan-2-yl)-éthyl]-pipérazin-1-yl]-urée,
• N-[4-(2-méthoxy-5-propyl-phényl)-thiazol-2-yl]-N'-[4-(2-pyrrolidin-1-yl-acétyl)-pipérazin-1-yl]-urée,
• N-[4-(2-méthoxy-5-propyl-phényl)-thiazol-2-yll-N'-[4-[2-(3-éthylamino-pyrrolidin-1-yl)-éthyl]-pipérazin-1-yl]-urée,
• N-[4-(5-cyclohexyl-2-méthoxy-phényl)-thiazol-2-yl]-N'-[4-(1-méthyl-pipéridin-4-yl)-pipérazin-1-yl]-urée,
• N-[4-(5-cyclohexyl-2-méthoxy-phény)-thiazol-2-yl]-N'-[4-(1-benzyl-pipéridin-3-yl)-pipérazin-1-yl]-urée,
• N-[4-(2-méthoxy-5-propyl-phényl)-thiazoi-2-y1]-N'-[4-(2-pyridin-4-yl-éthyl)-pipérazin-1-yl]-urée,
• N-[4-(2-méthoxy-5-pentafluoroéthyl-phényl)-thiazol-2-yl]-N'-(4-(2-pyrrolidin-1-yl-éthyl)-pipérazin-1-yl]-urée,
• N-[4-(5-cyclohexyl-2-méthoxy-phényl)-thiazol 2-yl]-N'-[4-(1-Isopropyl-pipéridin-3-yl)-pipérazin-1-yl]-urée,
• N-[4-(5-cyclohexyl-2-méthoxy-phényl)-thiazol-2-yl]-N'-[4-(2-pyrrolidin-1-yl-éthyl)-pipérazin-1-yl]-urée,
• N-[4-(5-cyclohexyl-2-méthoxy-phényl)-5-fluoro-thiazol-2-yl]-N'-[4-(1-méthyl-pipéridin-4-yl)-pipérazin-1-yl]-urée,
• N-[4-(5-cyclohexyl-2-méthoxy-phenyl)-thiazol-yl]-N'-[4-(2-oxo-2-pyrrolidin-1-yl-éthyl)-pipérazin-1-yl]-urée,
• N-[4-(5-cyclohexyl-2-éthoxy-phényl)-thiazol-2-yl]-N'-[4-(2-oxo-2-pyrrolidin-1-yl-éthyl)-pipérazin-1-yl]-urée,
• N-[4-(5-cyclohexyl-2-méthoxy-phényl)-thiazol-2-yl]-N'-[4-{2-oxo-2-morpholin-4-yl-éthyl)-pipérazin-1-yl]-urée,
• N-[4-(5-cyclohexyl-2-éthoxy-phénylthiazol-2-yl]-N'-4-(2-oxo-2-morpholino-4-yl-éthyl)-pipérazin-1-yl]-urée,
• (S) N-[4-(5-cyclohexyl-2-méthoxy-phényl)-thiazo)-2-yl]-N'-[4-(1-sulfomethyl-piperidin-3-yl-méthyt)-pipérazin-1-yl]-urée,
• N-[4-(5-cyclohexyl-2-méthoxy-phényl)-thiazol-2-yl]-N'-[4-(1-oxo-1-(1-oxo-pyridine-2yl-)meth-1-yl-piperidin-3-yl-méthyl)-pipérazin-1-yl]-urée,

6. Procédé de préparation d'un composé de formule (I) selon l'une quelconque des revendications 1 à 5, comprenant l'étape consistant à faire réagir un composé de formule (II) avec un dérivé amine de formule (III) 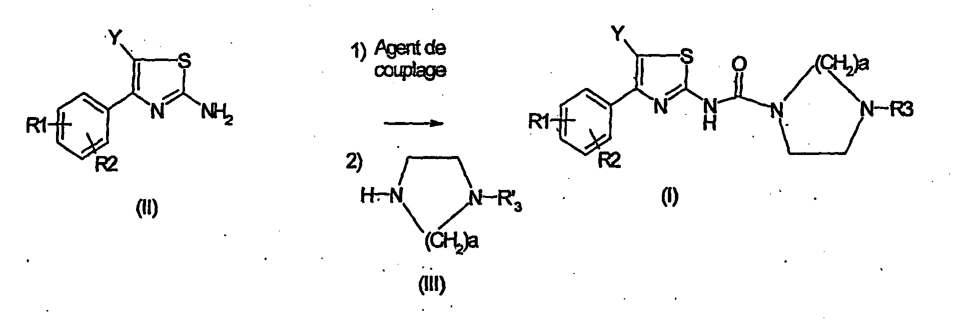 dans lesquelles R₁, R₂, Y, R₃ et a sont tels que définis dans la revendication 1 et R'₃ représente un groupe précurseur de R₃ ou un groupe R₃ tel que défini dans la revendication 1, en présence d'un agent de couplage et dans un solvant tel que le dichlorométhane, le diméthylformamide, le toluène, en présence d'une base telle que la triéthylamine, K₂CO₃, à une température variant de 0°C à 100°C.

7. Composé de formule (II.a) suivante: 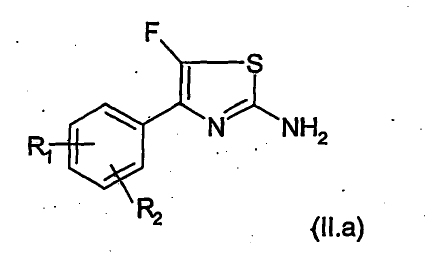 dans laquelle:
- R₁ représente un atome d'halogène, un groupe -O-(C₁-C₈)alkyle, -O-(C₃-C₁₀)cycloalkyl(C₁-C₈)alkyle ;
- R₂ représente un groupe -O-(C₁-C₈)alkyle, (C₁-C₈)alkyle, (C₃-C₁₀)cycloalkyle, trifluoro(C₁-C₄)alkyle, perfluoro(C₁-C₄)alkyle.

8. Médicament, **caractérisé en ce qu'**il comprend un composé de formule (I) selon l'une quelconque des revendication 1 à 5, ou un sel d'addition de ce composé à un acide pharmaceutiquement acceptable, ou encore un hydrate ou un solvat du composé de formule (I).

9. Composition pharmaceutique, **caractérisée en ce qu'**elle comprend un composé de formule (I) selon l'une quelconque des revendications 1 à 5, ou un sel pharmaceutiquement acceptable, un hydrate ou un solvat de ce composé, ainsi qu'au moins un excipent pharmaceutiquement acceptable:

10. Utilisation d'un composé de formule (1) selon l'une quelconque des revendications 1 à 5 pour la préparation d'un médicament destiné au traitement ou à la prévention des maladies et des syndromes immuno-inflammatoires aigus ou chroniques, tels que l'athérosclérose, des maladies allergiques ainsi que des maladies dans lesquels les processus angiogéniques sont impliqués.

11. Utilisation d'un composé de formule (I) selon la revendication 10 pour la préparation d'un médicament destiné au traitement ou à la prévention des cancers dans lesquels les processus angiogéniques sont impliqués.

12. Utilisation d'un composé de formule (I) selon l'une quelconque des revendications 1 à 5 pour la préparation d'un médicament destiné au traitement ou à la prévention des maladies virales ou bactériennes, les pathologies cardiaques, l'obésité.

13. Utilisation d'un composé de formule (I) selon l'une quelconque des revendications 1 à 5 pour la préparation d'un médicament destiné au traitement ou à la prévention des maladies liées à une modulation de l'activité du récepteur CCR2b.

## Claims

1. Compound of formula (I) below: 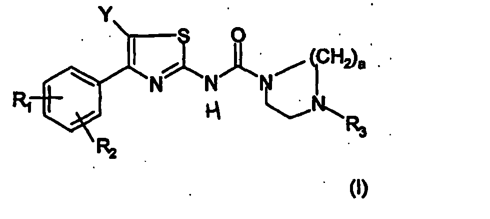 in which
- (i) R₁ is selected from the group consisting of H, halogen, (C₁-C₈)alkyl, trifluoro(C₁-C₄) alkyl, -OH, -O-(C₁-C₈)alkyl, -O-trifluoro (C₁-C₈) alkyl, -O-(C₃-C₁₀)cycloalkyl(C₁-C₈)alkyl, -O- (C₃-C₁₀) cycloalkyl, -O-CH₂-CH=CH₂ and (C₁-C₄)alkylthio;
- (ii) R₂ is selected from the group consisting of H, halogen, -OH, (C₁-C₈) alkyl, trifluoro(C₁-C₄)alkyl, perfluoro (C₁-C₄) alkyl, (C₃-C₁₀) cycloalkyl, -O- (C₁-C₈) alkyl, -O- (C₃-C₁₀) cycloalkyl (C₁-C₈) alkyl, -O- (C₃-C₁₀) cycloalkyl, -O-CH₂-CH=CH₂ and (C₃-C₈)cycloalkyl (C₁-C₈) alkyl;
- (iii) Y represents a hydrogen atom or a halogen;
- (iv) R₃ represents:
**a1)** a group of formula -(CH₂)ₚ-A
in which p represents 0, 1, 2, 3 or 4, and:
- when p represents 2, 3 or 4, A represents a group of formula: 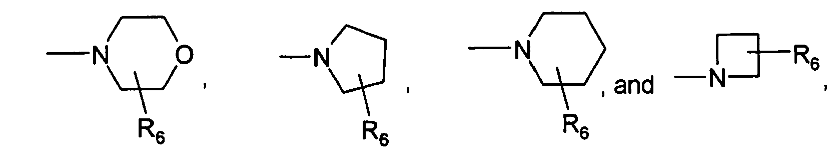 or -NR₄R₅,
in which R₆ is selected from the group consisting of H, F, (C₁-C₄) alkyl, - (CH₂)ₙOH, - (CH₂)ₙO (C₁-C₄) alkyl and - (CH₂)ₙNR₄R₅, where n represents 0, 1 or 2, and R₄ and R₅ represent, independently of one another, a hydrogen atom, or a (C₁-C₈) alkyl, -CO (C₁-C₄) alkyl or -CO-O-(C₁-C₈) alkyl group;
- or, when p represents 1, 2, 3 or 4, A represents a group of formula: 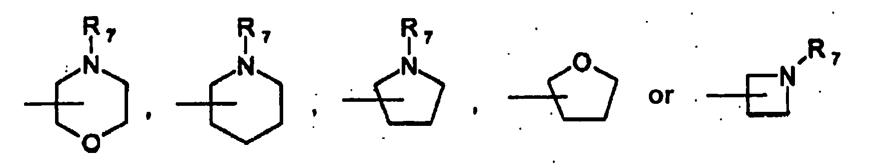 in which R₇ is selected from the group consisting of H, (C₁-C₈)alkyl, -CO- (C₁-C₈) alkyl, benzyl, -CO-O- (C₁-C₈)alkyl, -CO-O-benzyl, -CO-phenyl, -CO-heteroaryl, -CO-(C₃-C₁₀) cycloalkyl, -SO₂- (C₁-C₈) alkyl, -SO₂- (C₃-C₈) cycloalkyl and -SO₂-heteroaryl;
- or, when p represents 0, 1, 2, 3 or 4, A represents a group of formula: 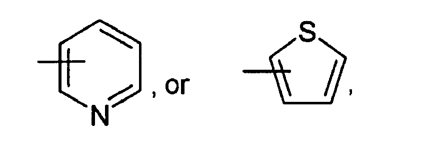 said group being optionally substituted with a (C₁-C₄) - alkyl group;
**a2)** a group of formula -(CH₂)ₚ-CO-A
in which p represents 1, 2, 3 or 4,
- A represents a group of formula: 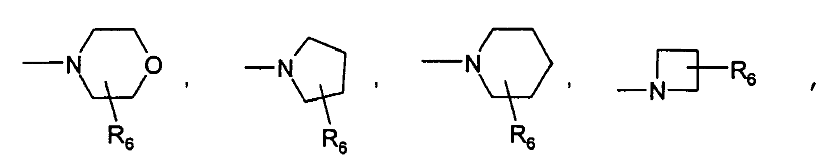 or -NR₄R₅,
in which R₄, R₅ and R₆ are as defined above;
**a3)** a group of formula -CO(CH₂)ₚ-A
in which p represents 0, 1, 2, 3 or 4
- when p represents 1, 2, 3 or 4, A represents a group of formula: 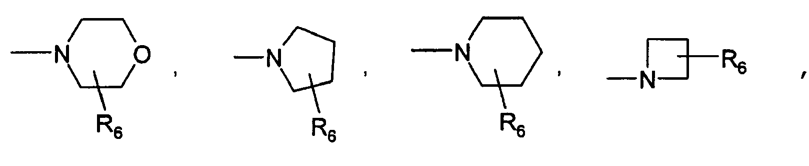 or -NR₄R₅,
in which R₄, R₅ and R₆ are as defined above;
- or, when p represents 0, 1, 2, 3 or 4, A represents a group of formula: 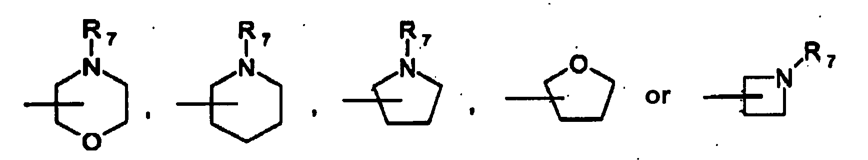 in which R₇ is as defined above;
- or, when p represents 0, 1, 2, 3 or 4, A represents a group of formula: 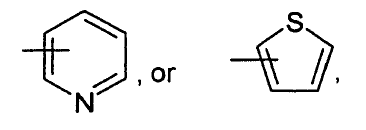 said group being optionally substituted with a (C₁-C₄) - alkyl group;
**a4)** a group -B
in which B represents a group of formula: 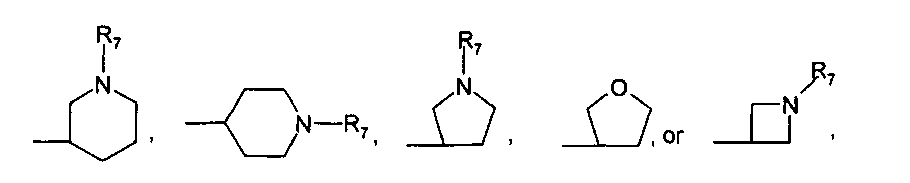 in which R₇ is as defined above;
- (v) a represents 2 or 3;
in the form of a base or of an addition salt with an acid, and also in the form of hydrates or of solvates.

2. Compound according to Claim 1, of formula (I.a) below: 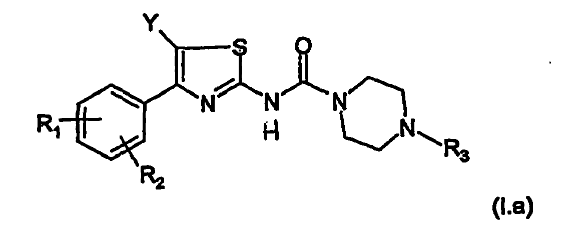 in which:
R₁, R₂, R₃ and Y are as defined above,
in the form of a base or of an addition salt with an acid, and also in the form of hydrates or of solvates.

3. Compound according to Claim 1, of formula (I.b) below: 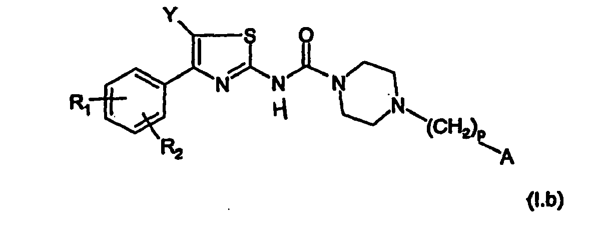 in which R₁, R₂, Y, p and A are as defined above,
in the form of a base or of an addition salt with an acid, and also in the form of hydrates or of solvates.

4. Compound according to Claim 1, of formula (I.c) below: 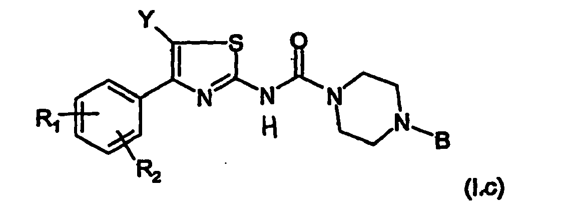 in which R₁, R₂, Y and B are as defined above,
in the form of a base or of an addition salt with an acid, and also in the form of hydrates or of solvates.

5. Compounds according to either one of Claims 1 and 2, chosen from:
• (R)-N-[4-(5-cyclohexyl-2-methoxyphenyl)thiazol-2-yl]-N'-[4-(1-methylpiperidin-3-ylmethyl)piperazin-1-yl]-urea,
• N-[4-(2-methoxy-5-propoxyphenyl)thiazol-2-yl]-N'-[4-(2-morpholin-4-ylethyl)piperazin-1-yl]urea,
• N-[4-(2-methoxy-5-propylphenyl)thiazol-2-yl]-N'-[4-(3-piperidin-1-ylpropyl)piperazin-1-yl]urea,
• N-[4-(2-methoxy-5-propylphenyl)thiazol-2-yl]-N'-[4-(2-dimethylaminoethyl)piperazin-1-yl]urea, N-[4-(2-methoxy-5-propylphenyl)thiazol-2-yl]-N'-[4-(2-oxo-2-pyrrolidin-1-ylethyl)piperazin-1-yl]urea,
• N-[4-(2-methoxy-5-propylphenyl)thiazol-2-yl]-N'-[4-(2-thiophen-2-ylethyl)piperazin-1-yl]urea,
• N-[4-(2-methoxy-5-propylphenyl)thiazol-2-yl]-N'-[4-[2-(tetrahydrofuran-2-yl)ethyl]piperazin-1-yl]urea,
• N-[4-(2-methoxy-5-propylphenyl)thiazol-2-yl]-N'-[4-(2-pyrrolidin-1-ylacetyl)piperazin-1-yl]urea,
• N-[4-(2-methoxy-5-propylphenyl)thiazol-2-yl]-N'-[4-[2-(3-ethylaminopyrrolidin-1-yl)ethyl]piperazin-1-yl]-urea,
• N-[4-(5-cyclohexyl-2-methoxyphenyl)thiazol-2-yl]-N'-[4-(1-methylpiperidin-4-yl)piperazin-1-yl]urea,
• N-[4-(5-cyclohexyl-2-methoxyphenyl)thiazol-2-yl]-N'-[4-(1-benzylpiperidin-3-yl)piperazin-1-yl]urea,
• N-[4-(2-methoxy-5-propylphenyl)thiazol-2-yl]-N'-[4-(2-pyridin-4-ylethyl)piperazin-1-yl]urea,
• N-[4-(2-methoxy-5-pentafluoroethylphenyl)thiazol-2-yl]-N'-[4-(2-pyrrolidin-1-ylethyl)piperazin-1-yl]urea,
• N-[4-(5-cyclohexyl-2-methoxyphenyl)thiazol-2-yl]-N'-[4-(1-isopropylpiperidin-3-yl)piperazin-1-yl]urea,
• N-[4-(5-cyclohexyl-2-methoxyphenyl)thiazol-2-yl]-N'-[4-(2-pyrrolidin-1-ylethyl)piperazin-1-yl]urea,
• N-[4-(5-cyclohexyl-2-methoxyphenyl)-5-fluorothiazol-2-yl]-N'-[4-(1-methylpiperidin-4-yl)piperazin-1-yl]-urea,
• N-[4-(5-cyclohexyl-2-methoxyphenyl)thiazol-2-yl]-N'-[4-(2-oxo-2-pyrrolidin-l-ylethyl)piperazin-l-yl]urea,
• N-[4-(5-cyclohexyl-2-ethoxyphenyl)thiazol-2-yl]-N'-[4-(2-oxo-2-pyrrolidin-1-ylethyl)piperazin-1-yl]urea,
• N-[4-(5-cyclohexyl-2-methoxyphenyl)thiazol-2-yl]-N'-[4-(2-oxo-2-morpholino-4-ylethyl)piperazin-1-yl]urea,
• N-[4-(5-cyclohexyl-2-ethoxyphenyl)thiazol-2-yl]-N'-[4-(2-oxo-2-morpholino-4-ylethyl)piperazin-1-yl]urea,
• (S) N-[4-(5-cyclohexyl-2-methoxyphenyl)thiazol-2-yl]-N'-[4-(1-sulphomethylpiperidin-3-ylmethyl)piperazin-1-yl]urea,
• N-[4-(5-cyclohexyl-2-methoxyphenyl)thiazol-2-yl]-N'-[4-(1-oxo-1-(1-oxopyridin-2-yl)meth-1-ylpiperidin-3-yl-methyl)piperazin-1-yl]urea.

6. Process for preparing a compound of formula (I) according to any one of Claims 1 to 5, comprising the step consisting in reacting a compound of formula (II) with an amine derivative of formula (III) 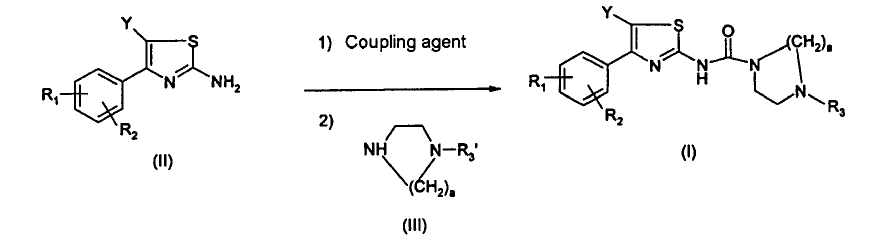 in which R₁, R₂, Y, R₃ and a are as defined in Claim 1 and R'₃ represents a precursor group of R₃ or a group R₃ as defined in Claim 1, in the presence of a coupling agent and in a solvent such as dichloromethane, dimethylformamide or toluene, in the presence of a base such as triethylamine or K₂CO₃, at a temperature ranging from 0°C to 100°C.

7. Compound of formula (II.a) below: 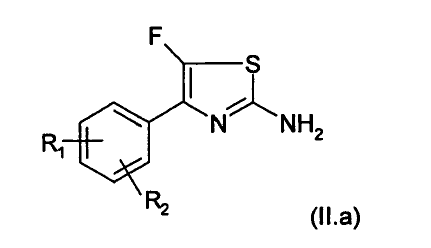 in which:
- R₁ represents a halogen atom, or an -O-(C₁-C₈)alkyl or -O-(C₃-C₁₀) cycloalkyl (C₁-C₈)alkyl group;
- R₂ represents an -O- (C₁-C₈) alkyl, (C₁-C₈) alkyl, (C₃-C₁₀) cycloalkyl, trifluoro(C₁-C₄)alkyl or perfluoro-(C₁-C₄) alkyl group.

8. Medicinal product, **characterized in that** it comprises a compound of formula (I) according to any one of Claims 1 to 5, or an addition salt of this compound with a pharmaceutically acceptable acid, or else a hydrate or a solvate of the compound of formula (I).

9. Pharmaceutical composition, **characterized in that** it comprises a compound of formula (I) according to any one of Claims 1 to 5, or a pharmaceutically acceptable salt, a hydrate or a solvate of this compound, and also at least one pharmaceutically acceptable excipient.

10. Use of a compound of formula (I) according to any one of Claims 1 to 5, for preparing a medicinal product intended for the treatment or prevention of acute or chronic immunoinflammatory diseases and syndromes, such as atherosclerosis, allergic diseases and also diseases in which angiogenic processes are involved.

11. Use of a compound of formula (I) according to Claim 10, for preparing a medicinal product intended for the treatment or prevention of cancers in which angiogenic processes are involved.

12. Use of a compound of formula (I) according to any one of Claims 1 to 5, for preparing a medicinal product intended for the treatment or prevention of viral or bacterial diseases, cardiac pathologies, or obesity.

13. Use of a compound of formula (I) according to any one of Claims 1 to 5, for preparing a medicinal product intended for the treatment or prevention of diseases associated with a modulation of the activity of the CCR2b receptor.

## Patentansprüche

1. Verbindung der folgenden Formel (I): 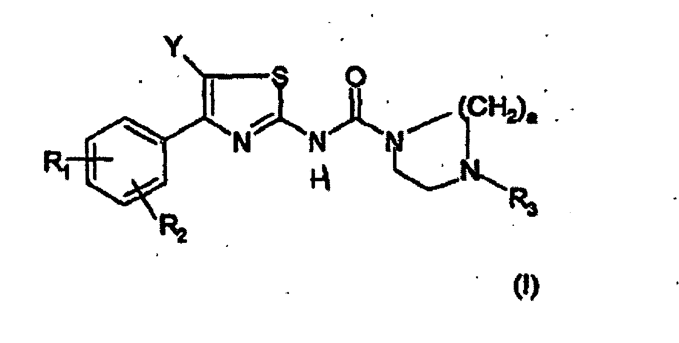 worin
- (i) R₁ ausgewählt ist aus der Gruppe bestehend aus H, Halogen, (C₁-C₈)-Alkyl, Trifluor- (C₁-C₄) -alkyl, -OH, -O- (C₁-C₈)-Alkyl, -O-Trifluor- (C₁-C₈) -alkyl, -O-(C₃-C₁₀) -Cycloalkyl- (C₁-C₈) -alkyl, -O-(C₃-C₁₀) -Cycloalkyl, -O-CH₂-CH=CH₂ und (C₁-C₄)-Alkylthio;
- (ii) R₂ ausgewählt ist aus der Gruppe bestehend aus H, Halogen, (C₁-C₈)-Alkyl, Trifluor- (C₁-C₄) -alkyl, Perfluor- (C₁-C₄)-alkyl, (C₃-C₁₀) -Cycloalkyl, -O- (C₁-C₈)-Alkyl, -O- (C₃-C₁₀) -Cycloalkyl- (C₁-C₈)-alkyl, -O- (C₃-C₁₀) -Cycloalkyl, -O-CH₂-CH=CH₂ und (C₃-C₈) -Cycloalkyl (C₁-C₈)-alkyl;
- (iii) Y für ein Wasserstoffatom oder ein Halogen steht;
- (iv) R₃ für:
a1) eine Gruppe der Formel -(CH₂)p-A steht,
worin p für 0, 1, 2, 3 oder 4 steht, und:
- wenn p für 2, 3 oder 4 steht, A für eine Gruppe der Formel: 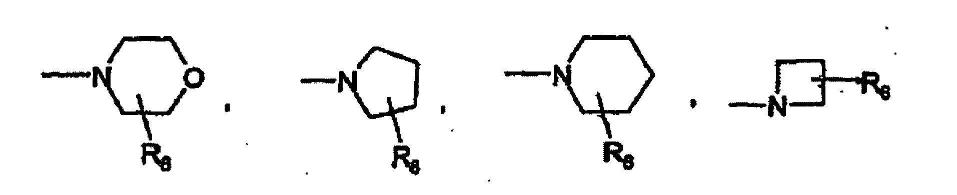 oder
-NR₉R₅ steht,
worin R₆ ausgewählt ist aus der Gruppe bestehend aus H, F, (C₁-C₄)-Alkyl, -(CH₂)ₙOH, -(CH₂)ₙO(C₁-C₄)-Alkyl und - (CH₂)ₙNR₄R₅, oder n für 0, 1 oder 2 steht und R₄ und R₅ unabhängig voneinander für ein Wasserstoffatom, eine (C₁-C₈) -Alkylgruppe, eine -CO (C₁-C₄) -Alkylgruppe oder eine -CO-O- (C₁-C₈)-Alkylgruppe stehen;
- oder, wenn p für 1, 2, 3 oder 4 steht, A für eine Gruppe der Formel: 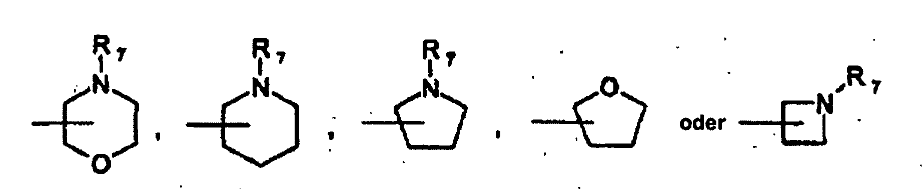 steht, worin R₇ ausgewählt ist aus der Gruppe bestehend aus H, (C₁-C₈) -Alkyl, -CO- (C₁-C₈) -Alkyl, Benzyl, -CO-O-(C₁-C₈)-Alkyl, -CO-O-Benzyl, -CO-Phenyl, -CO-Heteroaryl, -CO-(C₃-C₁₀)-Cycloalkyl, -SO₂-(C₁-C₈)-Alkyl, -SO₂- (C₃-C₈) -Cycloalkyl und -SO₂-Heteroaryl;
- oder, wenn p für 0, 1, 2, 3 oder 4 steht, A für eine Gruppe der Formel: 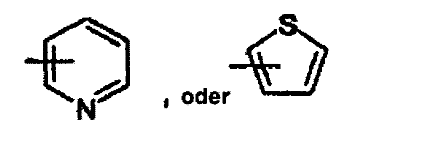 steht, wobei die Gruppe gegebenenfalls durch eine (Ci-C₄)-Alkylgruppe substituiert ist;
a2) eine Gruppe der Formel -(CH₂)p-CO-A steht,
worin p für 1, 2, 3 oder 4 steht,
- A für eine Gruppe der Formel: 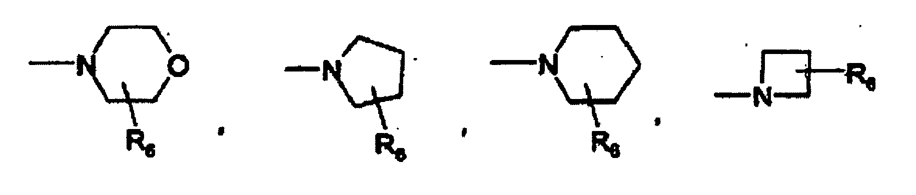 oder
-NR₄R₅ steht, worin R₄, R₅ und R₆ wie oben definiert sind;
a3) eine Gruppe der Formel -CO(CH₂)ₚ-A steht,
worin p für 0, 1, 2, 3 oder 4 steht,
- wenn p für 1, 2, 3 oder 4 steht, A für eine Gruppe der Formel: 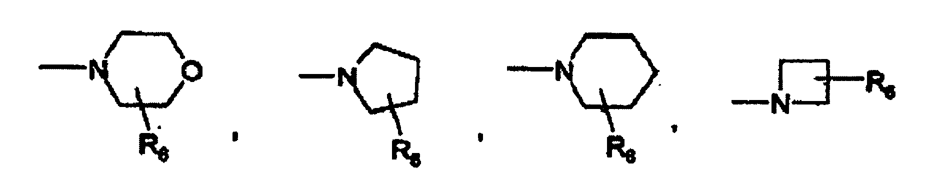 oder
-NR₄R₅ steht,
worin R₄, R₅ und R₆ wie oben definiert sind;
- oder, wenn p für 0, 1, 2, 3 oder 4 steht, A für eine Gruppe der Formel: 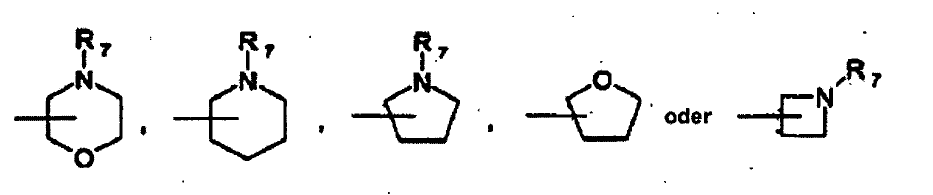 steht, worin R₇ wie zuvor definiert ist;
- oder, wenn p für 0, 1, 2, 3 oder 4 steht, A für eine Gruppe der Formel: 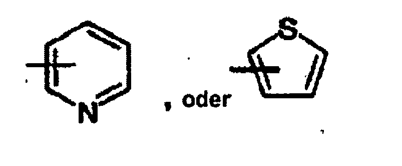 steht, wobei die Gruppe gegebenenfalls durch eine (C₁-C₄) -Alkylgruppe substituiert ist;
a4) eine Gruppe -B steht,
worin B für eine Gruppe der Formel: 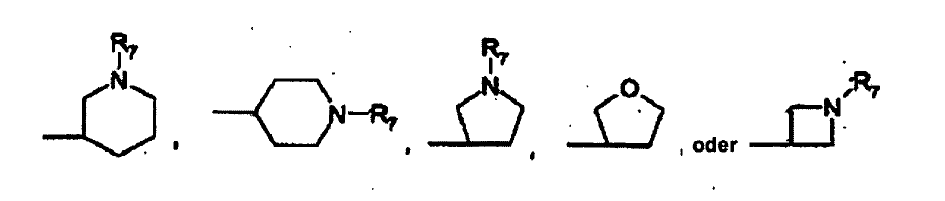 steht, worin R₇ wie zuvor definiert ist;
- (v) a für 2 oder 3 steht;
im Zustand einer Base oder eines Säureadditionsalzes, sowie im Zustand von Hydraten oder Solvaten.

2. Verbindung nach Anspruch 1, mit folgender Formel (I.a) : 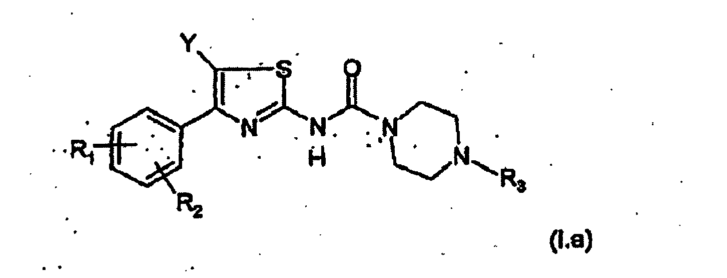 worin
R₁, R₂, R₃ und Y wie zuvor definiert sind,
im Zustand einer Base oder eines Säureadditionsalzes, sowie im Zustand von Hydraten oder Solvaten.

3. Verbindung nach Anspruch 1, mit folgender Formel (I.b) : 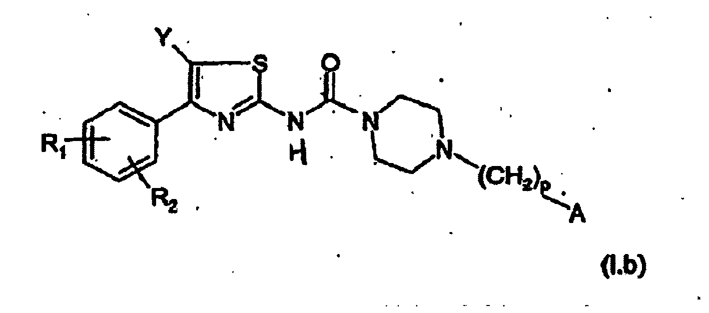 worin R₁, R₂, Y, p und A wie zuvor definiert sind,
im Zustand einer Base oder eines Säureadditionsalzes, sowie im Zustand von Hydraten oder Solvaten.

4. Verbindung nach Anspruch 1, mit folgender Formel (I.c) : 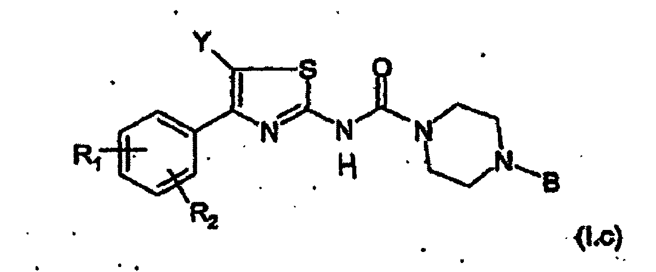 worin R₁, R₂, Y und B wie zuvor definiert sind,
im Zustand einer Base oder eines Säureadditionsalzes, sowie im Zustand von Hydraten oder Solvaten.

5. Verbindungen nach einem der Ansprüche 1 oder 2, ausgewählt aus:
• (R)-N- [4- (5-Cyclohexyl-2-methoxy-phenyl) -thiazol-2-yl]-N'-[4-(1-methyl-piperidin-3-yl-methyl)-piperazin-1-yl]-harnstoff,
• N-[4-(2-Methoxy-5-propoxy-phenyl)-thiazol-2-yl]-N'-[4-(2-morpholin-4-yl-ethyl)-piperazin-1-yl]-harnstoff,
• N-[4-(2-Methoxy-5-propyl-phenyl)-thiazol-2-yl]-N'-[4-(3-piperidin-1-yl-propyl)-piperazin-1-yl]-harnstoff,
• N-[4-(2-Methoxy-5-propyl-phenyl)-thiazol-2-yl]-N'-[4-(2-dimethylamino-ethyl)-piperazin-1-yl]-harnstoff,
• N-[4-(2-Methoxy-5-propyl-phenyl)-thiazol-2-yl]-N'-[4-(2-piperidin-1-yl-propyl)-piperazin-1-yl]-harnstoff,
• N-[4-(2-Methoxy-5-propyl-phenyl)-thiazol-2-yl]-N'-[4-(2-thiophen-2-yl-ethyl)-piperazin-1-yl]-harnstoff,
• N-[4-(2-Methoxy-5-propyl-phenyl)-thiazol-2-yl]-N'-[4-[2-(tetrahydro-furan-2-yl)-ethyl]-piperazin-1-yl]-harnstoff,
• N-[4-(2-Methoxy-5-propyl-phenyl)-thiazol-2-yl]-N'-[4-(2-pyrrolidin-1-yl-acetyl)-piperazin-1-yl]-harnstoff,
• N-[4-(2-Methoxy-5-propyl-phenyl)-thiazol-2-yl]-N'-[4-[2-(3-ethylamino-pyrrolidin-1-yl)-ethyl]-piperazin-1-yl]-harnstoff,
• N-[4-(5-Cyclohexyl-2-methoxy-phenyl)-thiazol-2-yl]-N'-[4-(1-methyl-piperidin-4-yl)-piperazin-1-yl]-harnstoff,
• N-[4-(5-Cyclohexyl-2-methoxy-phenyl-)thiazol-2-yl]-N'-[4-(1-benzyl-piperidin-3-yl)-piperazin-1-yl]-harnstoff,
• N-[4-(2-Methoxy-5-propyl]-phenyl)-thiazol-2-yl]-N'-[4-(2-pyridin-4-yl-ethyl)-piperazin-1-yl]-harnstoff,
• N-[4-(2-Methoxy-5-pentafluorethyl-phenyl)-thiazol-2-yl]-N'-[4-(2-pyrrolidin-1-yl-ethyl)-piperazin-1-yl]-harnstoff,
• N-[4-(5-Cyclohexyl-2-methoxy-phenyl)-thiazol-2-yl]-N'-[4-(1-isopropyl-piperidin-3-yl)-piperazin-1-yl]-harnstoff,
• N-[4-(5-Cyclohexyl-2-methoxy-phenyl)-thiazol-2-yl]-N'-[4-(2-pyrrolidin-1-yl-ethyl)-piperazin-1-yl]-harnstoff,
• N-[4-(5-Cyclohexyl-2-methoxy-phenyl)-5-fluorthiazol-2-yl]-N'-[4-(1-methyl-piperidin-4-yl)-piperazin-1-yl]-harnstoff,
• N-[4-(5-Cyclohexyl-2-methoxy-phenyl)-thiazol-2-yl]-N'-[4-(2-oxo-2-pyrrolidin-1-yl-ethyl)-piperazin-1-yl]-harnstoff,
• N-[4-(5-Cyclohexyl-2-ethoxy-phenyl)-thiazol-2-yl]-N'-[4-(2-oxo-2-pyrrolidin-1-yl-ethyl)-piperazin-1-yl]-harnstoff,
• N-[4-(5-Cyclohexyl-2-methoxy-phenyl)-thiazol-2-yl]-N'-[4-(2-oxo-2-morpholino-4-yl-ethyl)-piperazin-1-yl]-harnstoff,
• N-[4-(5-Cyclohexyl-2-ethoxy-phenyl)-thiazol-2-yl]-N'-[4-(2-oxo-2-morpholino-4-yl-ethyl)-piperazin-1-yl]-harnstoff,
• (S)-N-[4-(5-Cyclohexyl-2-methoxy-phenyl)-thiazol-2-yl]-N'-[4-(1-sulfomethyl-piperidin-3-yl-methyl)-piperazin-1-yl]-harnstoff,
• N-[4-(5-Cyclohexyl-2-methoxy-phenyl)-thiazol-2-yl]-N'-[4-(1-oxo-1-(1-oxo-pyridin-2-yl-)meth-1-yl-piperidin-3-yl-methyl)-piperazin-1-yl]-harnstoff.

6. Verfahren zur Herstellung einer Verbindung der Formel (I) nach einem der Ansprüche 1 bis 5, umfassend den Schritt, der darin besteht, eine Verbindung der Formel (II) mit einem Aminderivat der Formel (III) 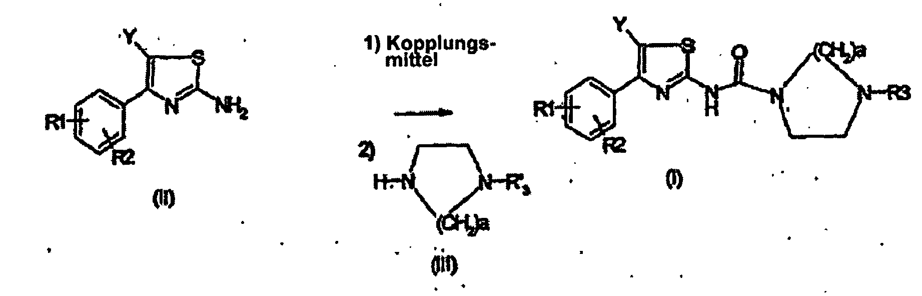 zur Reaktion zu bringen, worin R₁, R₂, Y, R₃ und a wie in Anspruch 1 definiert sind, und R'₃ für eine Vorläufergruppe von R₃ oder eine R₃-Gruppe steht, wie in Anspruch 1 definiert, in Gegenwart eines Kopplungsmittels und in einem Lösemittel, wie etwa Dichlormethan, Dimethylformamid, Toluol, in Gegenwart einer Base, wie etwa Triethylamin, K₂CO₃, bei einer Temperatur, die zwischen 0°C und 100°C variiert.

7. Verbindung der folgenden Formel (II.a): 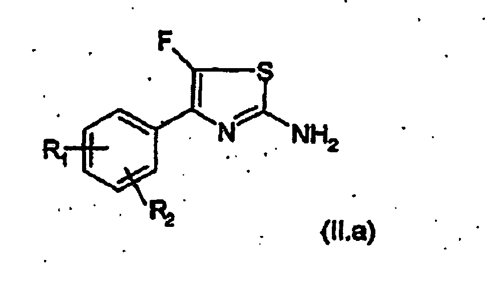 worin
- R₁ für ein Halogenatom, eine -O- (C₁-C₈) -Alkyl-, -O- (C₃-C₁₀) -Cycloalkyl- (C₁-C₈) -alkylgruppe steht;
- R₂ für eine -O-(C₁-C₈)-Alkyl-, (C₁-C₈) -Alkyl-, (C₃-C₁₀) -Cycloalkyl-, Trifluor (C₁-C₉) -alkyl-, Perfluor-(C₁-C₄)-alkylgruppe steht.

8. Medikament, **dadurch gekennzeichnet, dass** es eine Verbindung der Formel (I) nach einem der Ansprüche 1 bis 5 oder ein Additionssalz dieser Verbindung mit einer pharmazeutisch unbedenklichen Säure oder auch ein Hydrat oder ein Solvat der Verbindung der Formel (I) umfasst.

9. Pharmazeutische Zusammensetzung, **dadurch gekennzeichnet, dass** sie eine Verbindung der Formel (I) nach einem der Ansprüche 1 bis 5 oder ein pharmazeutisch unbedenkliches Salz, ein Hydrat oder ein Solvat dieser Verbindung sowie mindestens einen pharmazeutisch unbedenklichen Hilfsstoff umfasst.

10. Verwendung einer Verbindung der Formel (I) nach einem der Ansprüche 1 bis 5 zur Herstellung eines Medikaments, das zur Behandlung oder zur Vorbeugung von akuten oder chronischen immuninflammatorischen Krankheiten und Syndromen, wie etwa Atherosklerose, allergischen Krankheiten sowie Krankheiten, an denen angiogene Prozesse beteiligt sind, bestimmt ist.

11. Verwendung einer Verbindung der Formel (I) nach Anspruch 10 zur Herstellung eines Medikaments, das zur Behandlung oder zur Vorbeugung von Krebserkrankungen, an denen angiogene Prozesse beteiligt sind, bestimmt ist.

12. Verwendung einer Verbindung der Formel (I) nach einem der Ansprüche 1 bis 5 zur Herstellung eines Medikaments, das zur Behandlung oder zur Vorbeugung von viralen oder bakteriellen Krankheiten, kardialen Pathologien, Obesität bestimmt ist.

13. Verwendung einer Verbindung der Formel (I) nach einem der Ansprüche 1 bis 5 zur Herstellung eines Medikaments, das zur Behandlung oder zur Vorbeugung von Krankheiten bestimmt ist, die mit einer Modulation der Aktivität des Rezeptors CCR2b verbunden sind.
